# EUROPEAN PATENT APPLICATION

(11) **EP 4 089 172 A2**
(43) Date of publication of application: **16.11.2022**
(21) Application number: 22160356.6
(22) Date of filing: 03.03.2016
(51) Int. Cl.: C12N 15/86, C07K 14/705

(54) **MULTIPLE VECTOR SYSTEM AND USES THEREOF**

(30) Priority: 03.03.2015 US 201562127463 P
(62) Divisional of application: 16710113.8
(71) Applicant: Fondazione Telethon, 00185 Roma (IT)
(72) Inventor: COLELLA, Pasqualina, Pozzuoli 80078 (IT); AURICCHIO, Alberto, Pozzuoli 80078 (IT); TRAPANI, Ivana, Pozzuoli 80078 (IT)
(74) Representative: D Young & Co LLP

(57) **Abstract**

The present invention relates to constructs, vectors, relative host cells and pharmaceutical compositions which allow an effective gene therapy, in particular of genes larger than 5Kb.

## Description

### TECHNICAL FIELD

The present invention relates to constructs, vectors, relative host cells and pharmaceutical compositions which allow an effective gene therapy, in particular of genes larger than 5Kb.

### BACKGROUND OF THE INVENTION

Sight-restoring therapy for many inherited retinal degenerations (IRDs) is still a major unmet medical need. Gene therapy with adeno-associated viral (AAV) vectors represents, to date, the most promising approach for treatment of many IRDs. Indeed, years of pre-clinical research and a number of clinical trials for different IRDs have defined AAV's ability to efficiently deliver therapeutic genes to diseased retinal layers [photoreceptors (PR) and retinal pigment epithelium (RPE)]^{1,2} and have underlined their excellent safety and efficacy profiles in humans ³⁻⁷. Despite this, one of the main obstacles to expand this success to other blinding condition is the packaging capacity of AAV vectors (~5 kb). This has become a limiting factor for the development of gene replacement therapy for common IRDs due to mutations in genes with a coding sequence (CDS) larger than 5 kb (herein referred to also as large genes).

Therefore, considerable interest has been directed in recent years towards the identification of strategies to increase the carrying capacity of AAV. Dual AAV vectors, based on the ability of AAV genomes to concatamerize via intermolecular recombination, have been successfully exploited to address this issue ¹⁴⁻¹⁶. Dual AAV vectors are generated by splitting a large transgene expression cassette in two separate halves each packaged in a single normal size (NS; < 5 kb) AAV vector. The reconstitution of the full-length expression cassette is achieved upon co-infection of the same cell by both dual AAV vectors followed by either: i) inverted terminal repeat (ITR)-mediated tail-to-head concatemerization of the two vector genomes followed by splicing (dual AAV trans-splicing, TS)¹⁵, ii) homologous recombination between overlapping regions contained in the two vector genomes (dual AAV overlapping, OV)¹⁵, iii) a combination of the two (dual AAV hybrid)¹⁶. Others and the inventors have recently shown the potential of dual AAV vectors in the retina ^{14, 17-19}. The most used recombinogenic regions used in the context of dual AAV hybrid vectors derive from the 872 bp sequence of the middle one-third of the human alkaline phosphatase cDNA that has been shown to confer high levels of dual AAV hybrid vectors reconstitution ¹⁶. The inventors showed that dual AAV hybrid vectors including the AK sequence outperform those including the sense alkaline phosphatase head region sequence ¹⁴, which the inventors generated based on the description provided in Ghosh et al ²². Additional studies have shown that either the head or tail of this alkaline phosphatase region confers levels of transgene reconstitution similar to those achieved with the full-length middle one-third of the alkaline phosphatase cDNA ²². The inventors found that dual AAV trans-splicing and hybrid AK vectors (that contain the short AK recombinogenic sequence from the F1 phage) transduce efficiently the mouse and pig retina and rescue mouse models of Stargardt disease (STGD) and Usher 1B (USH1B) ^{14, 19}. The levels of PR transduction achieved with dual AAV TS and hybrid AK vectors resulted in significant improvement of the retinal phenotype of mouse models of IRDs and may be effective for treating inherited blinding conditions. Furthermore, vectors with heterologous ITR from serotypes 2 and 5 (ITR2 and ITR5, respectively), which are highly divergent (58% of homology 23), show both reduced ability to form circular monomers and increased directional tail-to-head concatamerization than vectors with homologous ITR ²⁴. Based on this, Yan et al have shown that dual AAV vectors with heterologous ITR2 and ITR5 reconstitute transgene expression more efficiently than dual AAV vectors with homologous ITR ^{24,25}.

Although these studies have highlighted the potential of dual AAV vectors for large gene reconstitution in the tissue of interest, such as the retina, they have also underlined critical issues that need to be addressed before considering further clinical translation of this strategy.

The production of truncated protein products from the 5'-half vector that contains the promoter sequence and/or from the 3'-half vector due to the low promoter activity of the ITR ^{14, 17, 20, 21} , still remains a major issue associated with the use of dual vectors . No formal toxicity studies have been so far performed to evaluate the potential detrimental effects of these truncated products *in vivo,* thus raising safety concern. Therefore, reduction or abolishment of their production is highly desirable. The present invention is thus aimed to solve this major issue associated with the use of dual vector systems.

### SUMMARY OF THE INVENTION

The present invention relates to constructs, vectors, relative host cells and pharmaceutical compositions which allow an effective gene therapy, in particular of genes larger than 5Kb. Large genes include, among others:

| **DISEASE** | **CAUSATIVE GENE** | **CELL AFFECTED** | **CDS SIZE (kb)** |
|---|---|---|---|
| USH1F | Protocadherin-related 15 (**PCDH15**) | Neurosensory retina | 5.9 |
| CSNB2 | Calcium channel, voltage-dependent, L type, alpha IF subunit **(CACNA1**) | Photoreceptors | 5.9 |
| ad RP | Small nuclear ribonucleoprotein 200 kDa (**SNRNP200**) | Photoreceptors and RPE | 6.4 |
| ad or ar RP | Retinitis pigmentosa 1 **(RP1)** | Photoreceptors | 6.5 |
| USH1B | Myosin IIVA (**MYO7A**) | Photoreceptors and RPE | 6.7 |
| STGD1 | ATP-binding cassette, sub-family A, member 4 (**ABCA4**) | Photoreceptors | 6.8 |
| ad RP | Pre-mRNA processing factor 8 homologue (**PRPF8**) | Photoreceptors and RPE | 7.0 |
| Occult macular dystrophy | Retinitis pigmentosa 1-like 1 **(RP1L1**) | Photoreceptors | 7.2 |
| LCA10 | Centrosomal protein 290 kDa (**CEP290**) | Photoreceptors | 7.5 |
| RP | **EYS** | Photoreceptors and extracellular matrix | 9,4 |
| USH1D | Cadherin 23 (**CDH23**) | Neurosensory retina | 10 |
| Alstrom Syndrome | **ALMS1** | Photoreceptors | 12,5 |
| USH2A and RP | Usherin (**USH2A**) | Neurosensory retina | 15.6 |
| ad macular dystrophy | Hemicentin 1 (**HMCN1)** | Photoreceptors and RPE | 17 |
| USH2C | G-coupled receptor 98 (**GPR98**) | Neurosensory retina | 18.9 |

Stargardt disease (STGD1; MIM#248200) is the most common form of inherited macular degeneration caused by mutations in *ABCA4* (CDS: 6822 bp), which encodes the photoreceptor-specific all-trans retinal transporter ^{8,9}. Cone-rod dystrophy type 3, fundus flavimaculatus, age-related macular degeneration type 2, Early-onset severe retinal dystrophy, and Retinitis pigmentosa type 19 are also associated with ABCA4 mutations (ABCA4-associated diseases). Usher syndrome type IB (USH1B; MIM#276900) is the most severe combined form of retinitis pigmentosa and deafness caused by mutations in *MYO7A* (CDS: 6648 bp)¹⁰, which encodes for an actin-based motor expressed in both PR and RPE within the retina ¹¹⁻¹³.

Furthermore, many other genetic diseases, not necessarily causing retinal symptoms, are due to mutations in large genes. These include, among others: Duchenne muscular dystrophy due to mutations in *DMD*, cystic fibrosis due to mutations in *CFTR,* hemophilia A due to mutations in F8 and dysferlinopathies due to mutations in the *DYSF* gene.

In particular, the present invention is aimed to decreasing expression of a truncated protein product associated with multiple vector systems, preferably with multiple viral vector systems, by use of signals that mediate the degradation of proteins or avoid their translation (hereinafter degradation signals). Degradation signals have never been used in the context of multiple viral vectors. In the present invention it was surprisingly found that when a degradation signal is present in at least one vector of a multiple vector system, expression of protein in truncated form is significantly decreased, leading to a higher yield of full length protein.

In a first aspect therefore the present invention provides a vector system to express the coding sequence of a gene of interest in a cell, said coding sequence comprising a first portion and a second portion, said vector system comprising:
a) a first vector comprising:
   - said first portion of said coding sequence (CDS1),
   - a first reconstitution sequence; and
b) a second vector comprising:
   - said second portion of said coding sequence (CDS2),
   - a second reconstitution sequence, wherein said first and second reconstitution sequences are selected from the group of:
      i] the first reconstitution sequence consists of the 3' end of said first portion of the coding sequence and the second reconstitution sequence consists of the 5'end of said second portion of the coding sequence, said first and second reconstitution sequences being overlapping sequences; or
      ii] the first reconstitution sequence comprises a splicing donor signal (SD) and the second reconstitution sequence comprises a splicing acceptor signal (SA), optionally each one of first and second reconstitution sequence further comprises a recombinogenic sequence,
   characterized by the fact that either one or both of the first and second vector further comprises a nucleotide sequence of a degradation signal said sequence being located in case of i) at the 3' end of the CDS1 and/or at the 5' end of the CDS2 and in case of ii) in 3' position relative to the SD and/or in 5' position relative to the SA.

Preferably both of the first and second vector further comprise said nucleotide sequence of a degradation signal, wherein the nucleotide sequence of the degradation signal in the first vector is identical to or differs from that in the second vector.

Preferably the first reconstitution sequence comprises a splicing donor signal (SD) and a recombinogenic region in 3' position relative to said SD, the second reconstitution sequence comprises a splicing acceptor signal (SA) and a recombinogenic sequence in 5' position relative to the SA; wherein said nucleotide sequence of a degradation signal is localized at the 5' end and/or at the 3' end of the nucleotide sequence of the recombinogenic region of either one or both of the first and second vector.

Preferably the nucleotide sequence of the degradation signal is selected from: one or more protein ubiquitination signals, one or more microRNA target sequences, and/or one or more artificial stop codons.

Preferably the nucleotide sequence of the degradation signal comprises or consists of a sequence encoding a sequence selected from CL1 SEQ ID No. 1, CL2 SEQ ID No. 2, CL6 SEQ ID No. 3, CL9 SEQ ID No. 4, CL10 SEQ ID No. 5, CL11 SEQ ID No. 6, CL12 SEQ ID No. 7, CL15 SEQ ID No. 8, CL16 SEQ ID No. 9, SL17 SEQ ID No. 10, or PB29 (SEQ ID No. 14 or SEQ ID No. 15); or wherein the nucleotide sequence of the degradation signal comprises or consists of a sequence selected from miR-204 SEQ ID No. 11, miR-124 SEQ ID No. 12 or miR-26a SEQ ID No. 13.

Preferably the nucleotide sequence of the degradation signal of the first vector comprises or consists of a sequence encoding CL1 SEQ ID No. 1 or comprises or consists of SEQ ID No. 16 or comprises or consists of miR-204 SEQ ID No. 11 and miR-124 SEQ ID No. 12, preferably comprises three copies of miR 204 SEQ ID No. 11 and three copies of miR 124 SEQ ID No. 12, or comprises or consists of miR-26a SEQ ID No. 13, preferably comprises four copies of miR-26a SEQ ID No. 13.

Preferably the nucleotide sequence of the degradation signal of the second vector comprises or consists of a sequence encoding PB29 (SEQ ID No. 14 or SEQ ID No. 15) or comprises or consists of SEQ ID No. 19 or SEQ ID No. 20, preferably the degradation signal of the second vector comprises or consists of a sequence encoding three copies of PB29 of SEQ ID No. 14 or SEQ ID No. 15.

Preferably the first vector further comprises a promoter sequence operably linked to the 5'end portion of said first portion of the coding sequence (CDS1).

Preferably both of the first vector and the second vector further comprise a 5'-terminal repeat (5'-TR) nucleotide sequence and a 3'-terminal repeat (3'-TR) nucleotide sequence, preferably the 5'-TR is a 5'-inverted terminal repeat (5'-ITR) nucleotide sequence and the 3'-TR is a 3'-inverted terminal repeat (3'-ITR) nucleotide sequence, preferably the ITRs derive from the same virus serotype or from different virus serotypes, preferably the virus is an AAV.

Preferably the recombinogenic sequence is selected from the group consisting of: AK GGGATTTTGCCGATTTCGGCCTATTGGTTAAAAAATGAGCTGATTTAACAAAAATTT AACGCGAATTTTAACAAAAT(SEQ ID No. 22) or GGGATTTTTCCGATTTCGGCCTATTGGTTAAAAAATGAGCTGATTTAACAAAAATTT AACGCGAATTTTAACAAAAT (SEQ ID NO. 23), AP1 (SEQ ID NO. 24), AP2 (SEQ ID NO. 25), and AP (SEQ ID NO. 26).

Preferably the coding sequence is split into the first portion and the second portion at a natural exon-exon junction.

Preferably the splicing donor signal comprises or consists essentially of a sequence that is at least 70%, 75%, 80%, 85%, 90%, 95% or 100 % identical to GTAAGTATCAAGGTTACAAGACAGGTTTAAGGAGACCAATAGAAACTGGGCTTGTC GAGACAGAGAAGACTCTTGCGTTTCT (SEQ ID No. 27).

Preferably the splicing acceptor signal comprises or consists essentially of a sequence that is at least 70%, 75%, 80%, 85%, 90%, 95% or 100 % identical to GATAGGCACCTATTGGTCTTACTGACATCCACTTTGCCTTTCTCTCCACAG (SEQ ID No. 28)

Preferably the first vector further comprises at least one enhancer nucleotide sequence, operably linked to the coding sequence.

Preferably the coding sequence encodes a protein able to correct a retinal degeneration. Preferably the coding sequence encodes a protein able to correct Duchenne muscular dystrophy, cystic fibrosis, hemophilia A and dysferlinopathies.

In case of retinal degradation, preferably the coding sequence is the coding sequence of a gene selected from the group consisting of: ABCA4, MYO7A, CEP290, CDH23, EYS, PCDH15, CACNA1, SNRNP200, RP1, PRPF8, RP1L1, ALMS1, USH2A, GPR98, HMCN1.

In case of Duchenne muscular dystrophy, cystic fibrosis, hemophilia A and dysferlinopathies, preferably the coding sequence is the coding sequence of a gene selected from the group consisting of: DMD, CFTR, F8 and DYSF.

Preferably the first vector does not comprise a poly-adenylation signal nucleotide sequence. Preferably the vector system comprises:
a) a first vector comprising in a 5'-3' direction:
   - a 5'-inverted terminal repeat (5'-ITR) sequence;
   - a promoter sequence;
   - a 5' end portion of a coding sequence of a gene of interest (CDS1), said 5'end portion being operably linked to and under control of said promoter;
   - a nucleotide sequence of a splicing donor signal;
   - a nucleotide sequence of a recombinogenic region; and
   - a 3'-inverted terminal repeat (3'-ITR) sequence; and
b) a second vector comprising in a 5'-3' direction:
   - a 5'-inverted terminal repeat (5'-ITR) sequence;
   - a nucleotide sequence of a recombinogenic region;
   - a nucleotide sequence of a splicing acceptor signal;
   - the 3'end of the coding sequence (CDS2);
   - a poly-adenylation signal nucleotide sequence; and
   - a 3'-inverted terminal repeat (3'-ITR) sequence,
   characterized by further comprising a nucleotide sequence of a degradation signal, said sequence being localized at 5' end or 3' end of the nucleotide sequence of the recombinogenic region of either one or both of the first and second vector.

Preferably in the vectors of the invention said first and second vector is independently a viral vector, preferably an adeno viral vector or adeno-associated viral (AAV) vector, preferably said first and second adeno-associated viral (AAV) vectors are selected from the same or different AAV serotypes, preferably the adeno-associated virus is selected from the serotype 2, the serotype 8, the serotype 5, the serotype 7 or the serotype 9.

Preferably the vector system of the invention further comprises a third vector comprising a third portion of said coding sequence (CDS3) and a reconstitution sequence, wherein the second vector comprises two reconstitution sequences, each reconstitution sequence located at each end of CDS2.

Prefererably the reconstitution sequence of the first vector consists of the 3' end of CDS1, the two reconstitution sequences of the second vector consist each respectively of the 5'end and of the 3' end of CDS2, the reconstitution sequence of the third vector consists of the 5' end of CDS3;
wherein said reconstitution sequence of the first vector and said reconstitution sequence of the second vector consisting of the 5'end of CDS2 are overlapping sequences, and
wherein said reconstitution sequence of the second vector consisting of the 3'end of CDS2 and said reconstitution sequence of said third vector are overlapping sequences;
wherein said second vector further comprises a degradation signal, said degradation signal being located at the 5' end and/or at the 3' end of the CDS2.

Preferably the third vector further comprises at least one nucleotide sequence of a degradation signal.

Preferably the second vector further comprises a poly-adenylation signal nucleotide sequence linked to the 3'end portion of said coding sequence (CDS2).

The present invention provides a host cell transformed with the vector system as defined above. Preferably the vector system or the host cell of the invention is for medical use. Preferably for use in gene therapy. Preferably for use in the treatment and/or prevention of a pathology or disease characterized by a retinal degeneration or for use in the prevention and/or treatment of Duchenne muscular dystrophy, cystic fibrosis, hemophilia A and dysferlinopathies.

Preferably the retinal degeneration is inherited.

Preferably the pathology or disease is selected from the group consisting of: retinitis pigmentosa (RP), Leber congenital amaurosis (LCA), Stargardt disease (STGD), Usher disease (USH), Alstrom syndrome, congenital stationary night blindness (CSNB), macular dystrophy, occult macular dystrophy, a disease caused by a mutation in the *ABCA4* gene.

The invention provides a pharmaceutical composition comprising the vector system or the host cell as defined above and pharmaceutically acceptable vehicle.

The invention provides a method for treating and/or preventing a pathology or disease characterized by a retinal degeneration comprising administering to a subject in need thereof an effective amount of the vector system, the host cell or the pharmaceutical composition as defined above.

The invention provides a method for treating and/or preventing Duchenne muscular dystrophy, cystic fibrosis, hemophilia A or dysferlinopathies comprising administering to a subject in need thereof an effective amount of the vector system, the host cell or the pharmaceutical composition as defined above.

The invention provides the use of a nucleotide sequence of a degradation signal in a vector system to decrease expression of a protein in truncated form.

The invention provides a method for decreasing expression of a protein in truncated form comprising inserting a nucleotide sequence of a degradation signal in one or more vector of a vector system.

According to preferred embodiments of the invention, the vector system to express the coding sequence of a gene of interest in a cell comprises two vectors, each vector comprising a different portion of said coding sequence and a reconstitution sequence; preferably, the reconstitution sequence of the first vector is a sequence comprising a splicing donor, while the reconstitution sequence of the second vector is a sequence comprising a splicing acceptor.

According to a further preferred embodiments of the invention, the vector system to express the coding sequence of a gene of interest in a cell comprises three vectors, each vector comprising a different portion of said coding sequence and at least one reconstitution sequence; preferably, the first vector comprises a reconstitution sequence comprising a splicing donor in 3' position relative to the first portion of the coding sequence, the second vector comprises a reconstitution sequence comprising a splicing acceptor in 5' position relative to the second portion coding sequence and a reconstitution sequence comprising a splicing donor in 3' position relative to the second portion of the coding sequence, the third vector comprises a reconstitution sequence comprising a splicing acceptor in 5' position relative to the third portion coding sequence. Preferably, the reconstitution sequences of the first and the second vector or the reconstitution sequences of the first, the second and the third vector further comprise a recombinogenic region, preferably located in 3' position relative to the splicing donor and in 5' position relative to the splicing acceptor.

Either one or two or all the vectors of the vector system of the invention further comprise a nucleotide sequence of a degradation signal.

Preferably, the first vector comprises a degradation signal. Preferably, the second vector comprises a degradation signal.

According to preferred embodiments of the invention, wherein the vectors comprise reconstitution sequences that comprise a recombinogenic region, a degradation signals is localized at the 5' end or at the 3' end of the sequence of said recombinogenic region. According to preferred embodiments of the invention, the vector system to express the coding sequence of a gene of interest in a cell comprises two vectors; the first vector of the vector system comprising in a 5'-3' direction:
- the 5'end portion of the coding sequence of a gene of interest,
- the nucleic acid sequence of a splicing donor signal,
- the nucleic acid sequence of a recombinogenic region, and
- the nucleic acid sequence of a degradation signal.

According to preferred embodiments of the invention, the vector system to express the coding sequence of a gene of interest in a cell comprises two vectors, the second vector of the vector system comprising in a 5'-3' direction:
- the nucleic acid sequence of the recombinogenic region,
- the nucleic acid sequence of the degradation signal,
- the nucleic acid sequence of the splicing acceptor signal, and
- the 3'end portion of the coding sequence of a gene of interest.

Preferably, the first vector of a vector system according to the invention further comprises a promoter sequence, more preferably said promoter sequence is operably linked to the 5'end of the first portion of the coding sequence of a gene of interest.

Preferably, the second vector of a vector system consisting of two vectors further comprises a poly-adenylation signal nucleic acid sequence, more preferably said poly-adenylation signal nucleic acid sequence is linked to the 3'end of the second portion of the coding sequence of a gene of interest. Preferably the first vector of a vector system according to the invention does not comprise a poly-adenylation signal nucleic acid sequence.

Preferably, the third vector of a vector system consisting of three vectors further comprises a poly-adenylation signal nucleic acid sequence, more preferably said poly-adenylation signal nucleic acid sequence is linked to the 3'end of the third portion of the coding sequence of a gene of interest.

Preferably, at least one of the vectors of the vector system of the invention, more preferably the first vector of the vector system of the invention, comprises a degradation signal of sequence comprising or consisting of a sequence encoding CL1 SEQ ID No. 1; preferably, said sequence encoding CL1 SEQ ID No. 1 comprises or consists of SEQ ID No. 16.

Preferably, at least one of the vectors of the vector system of the invention, more preferably the first vector of the vector system of the invention, comprises a degradation signal of sequence comprising miR-204 SEQ ID No. 11 and miR-124 SEQ ID No. 12, more preferably three copies of miR 204 SEQ ID No. 11 and three copies of miR 124 SEQ ID No. 12; preferably miR 204 sequence and miR 124 sequence and/or each copy of miR 204 sequence and of miR 124 sequence are linked by a linker sequence of at least 1, at least 2, at least 3, at least 4 nucleotides. Preferably, at least one of the vectors of the vector system of the invention, more preferably the first vector of the vector system of the invention, comprises a degradation signal of sequence comprising or consisting of miR-26a SEQ ID No. 13, more preferably comprising four copies of miR-26a SEQ ID No. 13.

Preferably, at least one of the vectors of the vector system of the invention, more preferably the second vector of the vector system of the invention, comprises a degradation signal of sequence comprising or consisting of a sequence encoding PB29 (SEQ ID No. 14 or SEQ ID No. 15); preferably, said sequence encoding PB29 comprises or consists of SEQ ID No. 19 or SEQ ID No. 20; still preferably, said degradation signal of sequence comprises or consists of a sequence encoding three copies of PB29 of SEQ ID No. 14 or SEQ ID No. 15.

According to a preferred embodiment of the invention, the vector system comprises:
a) a first vector comprising in a 5'-3' direction:
   - a 5'-inverted terminal repeat (5'-ITR) sequence;
   - a promoter sequence;
   - a first portion of a coding sequence of a gene of interest, preferably being the 5'end portion of said coding sequence, preferably said first portion being operably linked to and under control of said promoter;
   - a nucleic acid sequence of a splicing donor signal;
   - a nucleic acid sequence of a recombinogenic region; and
   - a 3'-inverted terminal repeat (3'-ITR) sequence; and
b) a second vector comprising in a 5'-3' direction:
   - a 5'-inverted terminal repeat (5'-ITR) sequence;
   - a nucleic acid sequence of a recombinogenic region;
   - a nucleic acid sequence of a splicing acceptor signal;
   - a second portion of a coding sequence of a gene of interest, preferably being the 3'end portion of said coding sequence;
   - a poly-adenylation signal nucleic acid sequence; and
   - a 3'-inverted terminal repeat (3'-ITR) sequence,
   said first and/or second vector further comprising a nucleic acid sequence of a degradation signal, said sequence being localized at the 5' end or 3' end of the nucleic acid sequence of the recombinogenic region.

According to a further preferred embodiment of the invention, the vector system comprises:
a) a first vector comprising in a 5'-3' direction:
   - a 5'-inverted terminal repeat (5'-ITR) sequence;
   - a promoter sequence;
   - a first portion of a coding sequence of a gene of interest, preferably being operably linked to and under control of said promoter;
   - a nucleic acid sequence of a splicing donor signal;
   - a nucleic acid sequence of a recombinogenic region; and
   - a 3'-inverted terminal repeat (3'-ITR) sequence;
b) a second vector comprising in a 5'-3' direction:
   - a 5'-inverted terminal repeat (5'-ITR) sequence;
   - a nucleic acid sequence of a recombinogenic region;
   - a nucleic acid sequence of a splicing acceptor signal;
   - a second portion of a coding sequence of a gene of interest;
   - a nucleic acid sequence of a splicing donor signal;
      -- a nucleic acid sequence of a recombinogenic region;
   - a 3'-inverted terminal repeat (3'-ITR) sequence; and
c) a third vector comprising in a 5'-3' direction:
   - a 5'-inverted terminal repeat (5'-ITR) sequence;
   - a nucleic acid sequence of a recombinogenic region;
   - a nucleic acid sequence of a splicing acceptor signal;
   - a third portion of a coding sequence of a gene of interest;
   - a poly-adenylation signal nucleic acid sequence; and
   - a 3'-inverted terminal repeat (3'-ITR) sequence,
   said first and/or second and /or third vector further comprising a nucleic acid sequence of a degradation signal, said sequence being localized at the 5' end or 3' end of the nucleic acid sequence of the recombinogenic region(s).

Preferably the pathology or disease is selected from: Usher type IF (USH1F), congenital stationary night blindness (CSNB2), autosomal dominant (ad) and/or autosomal recessive (ar) Retinitis Pigmentosa (RP), USH1B, STGD1, Leber Congenital Amaurosis type 10 (LCA10), RP, Usher type ID (USH1D), Usher type 2A (USH2A), autosomal dominant macular dystrophy, Usher type 2C (USH2C), Occult macular dystrophy, Alstrom Syndrome.

In the present invention the vector system means a construct system, a plasmid system and also viral particles.

In the present invention the construct or vector system may include more than two vectors.

In particular the construct system may include a third vector comprising a third portion of the sequence of interest.

In the present invention the full length coding sequence reconstitutes or is obtained when the various (2, 3 or more) vectors are introduced in the cell.

The coding sequence may be split in two. The portions may be equal or different in length. The full length coding sequence is obtained when the vectors of the vector system are introduced into the cell. The first portion may be the 5' end portion of the coding sequence. The second portion may be the 3'end of the coding sequence. Still, the coding sequence may be split in three portions. The portions may be equal or different in length. The full length coding sequence is obtained when the vectors of the vector system are introduced into the cell. The first portion being the 5' portion of a coding sequence, the second portion being a middle portion of the coding sequence, the third portion being the 3' portion of a coding sequence.

In the present invention the cell is preferably a mammal cell, preferably a human cell.

In the present invention the presence of one degradation signal in any of the vectors is sufficient to decrease the production of the protein in truncated form.

The term degradation signal means a sequence (either nucleotidic or amminoacidic), which can mediate the degradation of the mRNA/protein in which it is included.

The term "protein in truncated form" or a "truncated protein" is a protein which is not produced in its full-length form, since it presents deletions ranging from single to many aminoacids (as an example from 1 to 10, 1 to 20, 1 to 50, 100, 200, ect...).

In the present invention a "reconstitution sequence" is a sequence allowing for the reconstitution of the full length coding sequence with the correct frame, therefore allowing the expression of a functional protein.

The term "splicing donor/acceptor signal" means nucleotidic sequences involved in the splicing of the mRNA.

In the present invention any splicing donor or acceptor signal sequence from any intron may be used. The skilled person knows how to recognizes and select the appropriate splicing donor or acceptor signal sequence by routine experiments.

In the present invention two sequences are overlapping when at least a portion of each of said sequences is homologous one to the other. The sequences may be overlapping for at least 1, at least 2, at least 5, at least 10, at least 20, at least 50, at least 100 , at least 200 nucleotides.

The term "recombinogenic region or sequence" means a sequence which mediates the recombination between two different sequences. "Recombinogenic region or sequence" and "region of homology" are used herein interchangeably.

The term "terminal repeat" means sequences which are repeated at both ends of a nucleotide sequence.

The term "inverted terminal repeat" means sequences which are repeated at both ends of a nucleotide sequence in the opposite orientation (reverse complementary).

A protein ubiquitination signal is a signal that mediates protein degradation by the proteasome. In the present invention when a degradation signal comprises repeated sequences, being the same sequence or different sequences, said repeated sequences are preferably linked by a linker of at least 1 nucleotide.

An artificial stop codon is a nucleotide sequence purposely included in a transcript to induce the premature termination of the translation of a protein.

An enhancer sequence is a sequence that increases the transcription of a gene.

Suitable degradation signals, according to the present invention include: (i) the short degron CL1, a C-terminal destabilizing peptide that shares structural similarities with misfolded proteins and is thus recognized by the ubiquitination system ^{31, 32}, (ii) ubiquitin, whose fusion at the N-terminal of a donor protein mediates both direct protein degradation or degradation via the N-end rule pathway ^{33, 34} and (iii) the N-terminal PB29 degron which is a 9 aminoacid-long peptide which, similarly to the CL1 degron, is predicted to fold in structures that are recognized by enzymes of the ubiquitination pathway ³⁵. The inventors have found that inclusion of degradation sequences or signals in multiple vector systems mitigate the expression of truncated proteins. In one instance, the inventors have found that including a CL1 degradation signal results in the selective degradation of truncated proteins from the 5'-half without affecting full-length protein production both *in vitro* and in the large pig retina.

Additionally, artificial stop codons can be inserted to cause the early termination of an mRNA. MicroRNA (miR) target sequences, artificial stop codons or protein ubiquitination signals can be exploited to mediate the degradation of truncated protein products. In the present invention a degradation signal sequence can comprise repeated sequences, such as more than one microRNA (miR) target sequence, artificial stop codon or protein ubiquitination signal, said repeated sequences being the same sequence or different sequences repeated at least twice; preferably, the repeated sequences are linked by a linker of at least 1 nucleotide.

Among the miR expressed in the retina, miR-let7b or -26a are expressed at high levels ²⁶⁻²⁹ while miR-204 and -124 have been shown to restrict AAV-mediated transgene expression to either RPE or photoreceptors ³⁰. Karali et al³⁰ tested the efficacy of the miR target sites in modulating the expression of a gene included in a single AAV vector in specific cell types. In Karali et al, miR target sites were included in a canonical expression cassette (coding for the entire reporter gene), downstream of a coding sequence and before the polyadenylation signal (polyA). Karali et al used miR target sites for either miR-204 or miR-124 and used 4 tandem copies of each miR.

In the present invention miR may also be miR mimics (Xiao, et al. J Cell Physiol 212:285-292, 2007; Wang Z Methods Mol Biol 676:211-223, 2011*).* For the first time, the inventors applied these strategies to multiple vector constructs and were able to silence the expression of truncated proteins generated from such vectors.

During the past decade, gene therapy has been applied to the treatment of disease in hundreds of clinical trials. Various tools have been developed to deliver genes into human cells. In the present invention the delivery vehicles may be administered to a patient. A skilled worker would be able to determine appropriate dosage range. The term "administered" includes delivery by viral or non-viral techniques. Non-viral delivery mechanisms include but are not limited to lipid mediated transfection, liposomes, immunoliposomes, lipofectin, cationic facial amphiphiles (CFAs) and combinations thereof. Among viral delivery, genetically engineered viruses, including adeno-associated viruses, are currently amongst the most popular tool for gene delivery. The concept of virus-based gene delivery is to engineer the virus so that it can express the gene(s) of interest or regulatory sequences such as promoters and introns. Depending on the specific application and the type of virus, most viral vectors contain mutations that hamper their ability to replicate freely as wild-type viruses in the host. Viruses from several different families have been modified to generate viral vectors for gene delivery. These viruses include retroviruses, lentiviruses, adenoviruses, adeno-associated viruses, herpes viruses, baculoviruses, picornaviruses, and alphaviruses. The present invention preferably employs adeno-associated viruses. Most of the systems contain vectors that are capable of accommodating genes of interest and helper cells that can provide the viral structural proteins and enzymes to allow for the generation of vector-containing infectious viral particles. Adeno-associated virus is a family of viruses that differs in nucleotide and amino acid sequence, genome structure, pathogenicity, and host range. This diversity provides opportunities to use viruses with different biological characteristics to develop different therapeutic applications. As with any delivery tool, the efficiency, the ability to target certain tissue or cell type, the expression of the gene of interest, and the safety of adeno-associated viral-based systems are important for successful application of gene therapy. Significant efforts have been dedicated to these areas of research in recent years. Various modifications have been made to adeno-associated virus-based vectors and helper cells to alter gene expression, target delivery, improve viral titers, and increase safety. The present invention represents an improvement in this design process in that it acts to efficiently deliver genes of interest into such viral vectors.

An ideal adeno-associated virus-based vector for gene delivery must be efficient, cell-specific, regulated, and safe. The efficiency of delivery is important because it can determine the efficacy of the therapy. Current efforts are aimed at achieving cell-type-specific infection and gene expression with adeno-associated viral vectors. In addition, adeno-associated viral vectors are being developed to regulate the expression of the gene of interest, since the therapy may require long-lasting or regulated expression. Safety is a major issue for viral gene delivery because most viruses are either pathogens or have a pathogenic potential. It is important that during gene delivery, the patient does not also inadvertently receive a pathogenic virus that has full replication potential.

**Adeno-associated virus (AAV)** is a small virus which infects humans and some other primate species. AAV is not currently known to cause disease and consequently the virus causes a very mild immune response. Gene therapy vectors using AAV can infect both dividing and quiescent cells and persist in an extrachromosomal state without integrating into the genome of the host cell. These features make AAV a very attractive candidate for creating viral vectors for gene therapy, and for the creation of isogenic human disease models.

Wild-type AAV has attracted considerable interest from gene therapy researchers due to a number of features. Chief amongst these is the virus's apparent lack of pathogenicity. It can also infect non-dividing cells and has the ability to stably integrate into the host cell genome at a specific site (designated AAVS1) in the human chromosome 19. The feature makes it somewhat more predictable than retroviruses, which present the threat of a random insertion and of mutagenesis, which is sometimes followed by development of a cancer. The AAV genome integrates most frequently into the site mentioned, while random incorporations into the genome take place with a negligible frequency. Development of AAVs as gene therapy vectors, however, has eliminated this integrative capacity by removal of the *rep* and *cap* from the DNA of the vector. The desired gene together with a promoter to drive transcription of the gene is inserted between the ITRs that aid in concatamer formation in the nucleus after the single-stranded vector DNA is converted by host cell DNA polymerase complexes into double-stranded DNA. AAV-based gene therapy vectors form episomal concatamers in the host cell nucleus. In non-dividing cells, these concatemers remain intact for the life of the host cell. In dividing cells, AAV DNA is lost through cell division, since the episomal DNA is not replicated along with the host cell DNA. Random integration of AAV DNA into the host genome is detectable but occurs at very low frequency. AAVs also present very low immunogenicity, seemingly restricted to generation of neutralizing antibodies, while they induce no clearly defined cytotoxic response. This feature, along with the ability to infect quiescent cells present their dominance over adenoviruses as vectors for the human gene therapy.

### AAV genome, transcriptome and proteome

The AAV genome is built of single-stranded deoxyribonucleic acid (ssDNA), either positive- or negative-sensed, which is about 4.7 kilobase long. The genome comprises inverted terminal repeats (ITRs) at both ends of the DNA strand, and two open reading frames (ORFs): *rep* and *cap.* The former is composed of four overlapping genes encoding Rep proteins required for the AAV life cycle, and the latter contains overlapping nucleotide sequences of capsid proteins: VP1, VP2 and VP3, which interact together to form a capsid of an icosahedral symmetry.

### ITR sequences

The Inverted Terminal Repeat (ITR) sequences received their name because of their symmetry, which was shown to be required for efficient multiplication of the AAV genome. Another property of these sequences is their ability to form a hairpin, which contributes to so-called self-priming that allows primase-independent synthesis of the second DNA strand. The ITRs were also shown to be required for both integration of the AAV DNA into the host cell genome (19th chromosome in humans) and rescue from it, as well as for efficient encapsidation of the AAV DNA combined with generation of a fully assembled, deoxyribonuclease-resistant AAV particles.

With regard to gene therapy, ITRs seem to be the only sequences required *in cis* next to the therapeutic gene: structural (*cap*) and packaging (*rep*) genes can be delivered *in trans.* With this assumption many methods were established for efficient production of recombinant AAV (rAAV) vectors containing a reporter or therapeutic gene. However, it was also published that the ITRs are not the only elements required *in cis* for the effective replication and encapsidation.

A few research groups have identified a sequence designated *cis-acting Rep-dependent element* (CARE) inside the coding sequence of the *rep* gene. CARE was shown to augment the replication and encapsidation when present *in cis.*

As of 2006 there have been 11 AAV serotypes described, the 11th in 2004. All of the known serotypes can infect cells from multiple diverse tissue types. Tissue specificity is determined by the capsid serotype and pseudotyping of AAV vectors to alter their tropism range will likely be important to their use in therapy.

The inverted terminal repeat (ITR) sequences used in an AAV vector system of the present invention can be any AAV ITR. The ITRs used in an AAV vector can be the same or different. For example, a vector may comprise an ITR of AAV serotype 2 and an ITR of AAV serotype 5. In one embodiment of a vector of the invention, an ITR is from AAV serotype 2, 4, 5, or 8. In the present invention ITRs of AVV serotype 2 and serotype 5 are preferred. AAV ITR sequences are well known in the art (for example, see for ITR2, GenBank Accession Nos. AF043303.1 ; NC_001401.2; J01901.1 ; JN898962.1; see for ITR5, GenBank Accession No. NC_006152.1).

### Serotype 2

Serotype 2 (AAV2) has been the most extensively examined so far. AAV2 presents natural tropism towards skeletal muscles, neurons, vascular smooth muscle cells and hepatocytes.

Three cell receptors have been described for AAV2: heparan sulfate proteoglycan (HSPG), a_{V}β₅ integrin and fibroblast growth factor receptor 1 (FGFR-1). The first functions as a primary receptor, while the latter two have a co-receptor activity and enable AAV to enter the cell by receptor-mediated endocytosis. These study results have been disputed by Qiu, Handa, *et al..* HSPG functions as the primary receptor, though its abundance in the extracellular matrix can scavenge AAV particles and impair the infection efficiency.

### Serotype 2 and cancer

Studies have shown that serotype 2 of the virus (AAV-2) apparently kills cancer cells without harming healthy ones. "Our results suggest that adeno-associated virus type 2, which infects the majority of the population but has no known ill effects, kills multiple types of cancer cells yet has no effect on healthy cells," said Craig Meyers, a professor of immunology and microbiology at the Penn State College of Medicine in Pennsylvania. This could lead to a new anti-cancer agent.

### Other Serotypes

Although AAV2 is the most popular serotype in various AAV-based research, it has been shown that other serotypes can be more effective as gene delivery vectors. For instance AAV6 appears much better in infecting airway epithelial cells, AAV7 presents very high transduction rate of murine skeletal muscle cells (similarly to AAV1 and AAV5), AAV8 is superb in transducing hepatocytes and photoreceptors and AAV1 and 5 were shown to be very efficient in gene delivery to vascular endothelial cells. In the brain, most AAV serotypes show neuronal tropism, while AAV5 also transduces astrocytes. AAV6, a hybrid of AAV1 and AAV2, also shows lower immunogenicity than AAV2.

Serotypes can differ with the respect to the receptors they are bound to. For example AAV4 and AAV5 transduction can be inhibited by soluble sialic acids (of different form for each of these serotypes), and AAV5 was shown to enter cells via the platelet-derived growth factor receptor. The subject invention also concerns a viral vector system comprising a polynucleotide, expression construct, or vector construct of the invention. In one embodiment, the viral vector system is an AAV system. Methods for preparing viruses and virions comprising a heterologous polynucleotide or construct are known in the art. In the case of AAV, cells can be coinfected or transfected with adenovirus or polynucleotide constructs comprising adenovirus genes suitable for AAV helper function. Examples of materials and methods are described, for example, in U.S. Patent Nos. 8,137,962 and 6,967,018. An AAV virus or AAV vector of the invention can be of any AAV serotype, including, but not limited to, serotype AAV1 , AAV2, AAV3, AAV4, AAV5, AAV6, AAV7, AAV8, AAV9, AAV10, and AAV11. In a specific embodiment, an AAV2 or an AAV5 or an AAV7 or an AAV8 or an AAV9 serotype is utilized. In one embodiment, the AAV serotype provides for one or more tyrosine to phenylalanine (Y-F) mutations on the capsid surface. In a specific embodiment, the AAV is an AAV8 serotype having a tyrosine to phenylalanine mutation at position 733 (Y733F).

The delivery of one or more therapeutic genes or regulatory sequences such as promoters or introns by a vector system according to the present invention may be used alone or in combination with other treatments or components of the treatment.

The subject invention also concerns a host cell comprising the construct system or the viral vector system of the invention. The host cell can be a cultured cell or a primary cell, i.e., isolated directly from an organism, e.g., a human. The host cell can be an adherent cell or a suspended cell, i.e., a cell that grows in suspension. Suitable host cells are known in the art and include, for instance, DH5α, E. coli cells, Chinese hamster ovarian cells, monkey VERO cells, COS cells, HEK293 cells, and the like. The cell can be a human cell or from another animal. In one embodiment, the cell is a photoreceptor cell or an RPE cell. In a specific embodiment, the cell is a cone cell. The cell may also be a muscle cell, in particular a skeletal muscle cell, a lung cell, a pancreas cell, a liver cell, a kidney cell, an intestine cell, a blood cell. In a specific embodiment, the cell is a human cone cell or rod cell. The selection of an appropriate host is deemed to be within the scope of those skilled in the art from the teachings herein. Preferably, said host cell is an animal cell, and most preferably a human cell. The cell can express a nucleotide sequence provided in the viral vector system of the invention.

The man skilled in the art is well aware of the standard methods for incorporation of a polynucleotide or vector into a host cell, for example transfection, lipofection, electroporation, microinjection, viral infection, thermal shock, transformation after chemical permeabilisation of the membrane or cell fusion. The construct or vector system of the invention can also be introduced in vivo as naked DNA using methods known in the art, such as transfection, microinjection, electroporation, calcium phosphate precipitation, and by biolistic methods.

As used herein, the term "host cell or host cell genetically engineered" relates to host cells which have been transduced, transformed or transfected with the construct system or with the viral vector system of the invention

As used herein, the terms "nucleic acid" and "polynucleotide sequence" and "construct" refer to a deoxyribonucleotide or ribonucleotide polymer in either single- or double-stranded form, and unless otherwise limited, would encompass known analogs of natural nucleotides that can function in a similar manner as naturally-occurring nucleotides. The polynucleotide sequences include both full-length sequences as well as shorter sequences derived from the full-length sequences. It is understood that a particular polynucleotide sequence includes the degenerate codons of the native sequence or sequences which may be introduced to provide codon preference in a specific host cell. The polynucleotide sequences falling within the scope of the subject invention further include sequences which specifically hybridize with the sequences coding for a peptide of the invention. The polynucleotide includes both the sense and antisense strands as either individual strands or in the duplex.

The subject invention also contemplates those polynucleotide molecules having sequences which are sufficiently homologous with the polynucleotide sequences of the invention so as to permit hybridization with that sequence under standard stringent conditions and standard methods (Maniatis, T. et al, 1982).

The subject invention also concerns a construct system that can include regulatory elements that are functional in the intended host cell in which the construct is to be expressed. A person of ordinary skill in the art can select regulatory elements for use in appropriate host cells, for example, mammalian or human host cells. Regulatory elements include, for example, promoters, transcription termination sequences, translation termination sequences, enhancers, signal peptides, degradation signals and polyadenylation elements. A construct of the invention can comprise a promoter sequence operably linked to a nucleotide sequence encoding a desired polypeptide.

Promoters contemplated for use in the subject invention include, but are not limited to, native gene promoters, cytomegalovirus (CMV) promoter (KF853603.1, bp 149-735), chimeric CMV/chicken beta-actin promoter (CBA) and the truncated form of CBA (smCBA) promoter (US8298818 and Light-Driven Cone Arrestin Translocation in Cones of Postnatal Guanylate Cyclase-1 Knockout Mouse Retina Treated with AAVGC1), Rhodopsin promoter (NG_009115, bp 4205-5010), Interphotoreceptor retinoid binding protein promoter (NG_029718.1, bp 4777-5011), vitelliform macular dystrophy 2 promoter (NG_009033.1, bp 4870-5470), PR-specific human G protein-coupled receptor kinase 1 (hGRK1; AY327580.1 bp1793-2087 or bp 1793-1991) (Haire et al. 2006; U.S. Patent No. 8,298,818). However any suitable promoter known in the art may be used. In a specific embodiment, the promoter is a CMV or hGRK1 promoter. In one embodiment, the promoter is a tissue- specific promoter that shows selective activity in one or a group of tissues but is less active or not active in other tissue. In one embodiment, the promoter is a photoreceptor- specific promoter. In a further embodiment, the promoter is a cone cell-specific and/or rod cell-specific promoter.

Preferred promoters are CMV, GRK1, CBA and IRBP promoters. Still preferred promoters are hybrid promoter which combine regulatory elements from various promoters (as example the chimeric CBA promoter which combines an enhancer from the CMV promoter, the CBA promoter and the Sv40 chimeric intron, herein called CBA hybrid promoter.

Promoters can be incorporated into a construct using standard techniques known in the art. Multiple copies of promoters or multiple promoters can be used in a vector of the invention. In one embodiment, the promoter can be positioned about the same distance from the transcription start site as it is from the transcription start site in its natural genetic environment. Some variation in this distance is permitted without substantial decrease in promoter activity. In the system of the invention a transcription start site is typically included in the 5' construct but not in the 3' construct. In further embodiment a transcription start site may be included in the 3'construct upstream of the degradation signal.

A construct of the invention may optionally contain a transcription termination sequence, a translation termination sequence, signal peptide sequence, internal ribosome entry sites (IRES), enhancer elements, and/or post-trascriptional regulatory elements such as the Woodchuck hepatitis virus (WHV) posttranscriptional regulatory element (WPRE). Transcription termination regions can typically be obtained from the 3' untranslated region of a eukaryotic or viral gene sequence. Transcription termination sequences can be positioned downstream of a coding sequence to provide for efficient termination. In the system of the invention a transcription termination site is typically included in the 3' construct but not in the 5' construct.

Signal peptide sequence is an amino terminal sequence that encodes information responsible for the relocation of an operably linked polypeptide to a wide range of post-translational cellular destinations, ranging from a specific organelle compartment to sites of protein action and the extracellular environment. Enhancers are cis-acting elements that increase gene transcription and can also be included in a vector. Enhancer elements are known in the art, and include, but are not limited to, the CaMV 35S enhancer element, cytomegalovirus (CMV) early promoter enhancer element, and the SV40 enhancer element. DNA sequences which direct polyadenylation of the mRNA encoded by the structural gene can also be included in a vector. Preferably, in the present invention, the coding sequence is split into a first and a second fragment or portion (5' end portion and 3' end portion) at a natural exon-exon junction. Preferably each fragment or portion of the coding sequence should not exceed a size of 60 kb, preferably each fragment or portion of the coding sequence should not exceed a size of 50 Kb, 40 Kb, 30 Kb, 20 Kb, 10 Kb . Preferably each fragment or portion of the coding sequence may have a size of about 2Kb, 2.5Kb, 3Kb, 3.5Kb, 4Kb, 4.5Kb, 5Kb, 5.5 Kb, 6Kb, 6.5 Kb, 7kb, 7.5 Kb, 8 Kb, 8.5 Kb, 9Kb, 9.5 Kb or a smaller size.

Spliceosomal introns often reside within the sequence of eukaryotic protein-coding genes. Within the intron, a donor site (5' end of the intron), a branch site (near the 3' end of the intron) and an acceptor site (3' end of the intron) are required for splicing. The splice donor site includes an almost invariant sequence GU at the 5' end of the intron, within a larger, less highly conserved region. The splice acceptor site at the 3' end of the intron terminates the intron with an almost invariant AG sequence. Upstream (5'-ward) from the AG there is a region high in pyrimidines (C and U), or polypyrimidine tract. Upstream from the polypyrimidine tract is the branchpoint, which includes an adenine nucleotide. The spicing acceptor signal and the splicing donor signal may also be chosen by the skilled person in the art among sequences known in the art.

Signals that mediate the degradation of proteins and that have never been used before in the context of a multiple viral system include but are not limited to: short degrons as CL1, CL2, CL6, CL9, CL10, CL11, CL12, CL15, CL16, SL17, a C-terminal destabilizing peptide that shares structural similarities with misfolded proteins and is thus recognized by the ubiquitination system, ubiquitin, whose fusion at the N-terminal of a donor protein mediates both direct protein degradation or degradation via the N-end rule pathway, the N-terminal PB29 degron which is a 9 aminoacid-long peptide which, similarly to the CL1 degron, is predicted to fold in structures that are recognized by enzymes of the ubiquitination pathway, artificial stop codons that cause the early termination of an mRNA, microRNA (miR) target sequences.

As those skilled in the art can readily appreciate, there can be a number of variant sequences of a protein found in nature, in addition to those variants that can be artificially created by the skilled artisan in the lab. The polynucleotides and polypeptides of the subject invention encompasses those specifically exemplified herein, as well as any natural variants thereof, as well as any variants which can be created artificially, so long as those variants retain the desired functional activity. Also within the scope of the subject invention are polypeptides which have the same amino acid sequences of a polypeptide exemplified herein except for amino acid substitutions, additions, or deletions within the sequence of the polypeptide, as long as these variant polypeptides retain substantially the same relevant functional activity as the polypeptides specifically exemplified herein. For example, conservative amino acid substitutions within a polypeptide which do not affect the function of the polypeptide would be within the scope of the subject invention. Thus, the polypeptides disclosed herein should be understood to include variants and fragments, as discussed above, of the specifically exemplified sequences. The subject invention further includes nucleotide sequences which encode the polypeptides disclosed herein. These nucleotide sequences can be readily constructed by those skilled in the art having the knowledge of the protein and amino acid sequences which are presented herein. As would be appreciated by one skilled in the art, the degeneracy of the genetic code enables the artisan to construct a variety of nucleotide sequences that encode a particular polypeptide or protein. The choice of a particular nucleotide sequence could depend, for example, upon the codon usage of a particular expression system or host cell. Polypeptides having substitution of amino acids other than those specifically exemplified in the subject polypeptides are also contemplated within the scope of the present invention. For example, non-natural amino acids can be substituted for the amino acids of a polypeptide of the invention, so long as the polypeptide having substituted amino acids retains substantially the same activity as the polypeptide in which amino acids have not been substituted. Examples of non-natural amino acids include, but are not limited to, ornithine, citrulline, hydroxyproline, homoserine, phenylglycine, taurine, iodotyrosine, 2,4-diaminobutyric acid, a-amino isobutyric acid, 4-aminobutyric acid, 2- amino butyric acid, γ-amino butyric acid, ε-amino hexanoic acid, 6-amino hexanoic acid, 2-amino isobutyiic acid, 3 - amino propionic acid, norleucine, norvaline, sarcosine, homocitrulline, cysteic acid, τ-butylglycine, τ-butylalanine, phenylglycine, cyclohexylalanine, β-alanine, fluoro-amino acids, designer amino acids such as β-methyl amino acids, C-methyl amino acids, N-methyl amino acids, and amino acid analogues in general. Non-natural amino acids also include amino acids having derivatized side groups. Furthermore, any of the amino acids in the protein can be of the D (dextrorotary) form or L (levorotary) form. Amino acids can be generally categorized in the following classes: non-polar, uncharged polar, basic, and acidic. Conservative substitutions whereby a polypeptide having an amino acid of one class is replaced with another amino acid of the same class fall within the scope of the subject invention so long as the polypeptide having the substitution still retains substantially the same biological activity as a polypeptide that does not have the substitution. Table 1 provides a listing of examples of amino acids belonging to each class.

| Table 1. | |
|---|---|
| Class of Amino Acid | Examples of Amino Acids |
| Nonpolar | Ala, Val, Leu, Ile, Pro, Met. Phe, Trp |
| Uncharged Polar | Gly, Ser, Thr, Cys. Tyr, Asn, Gln |
| Acidic | Asp, Glu |
| Basic | Lys, Arg. His |

Also within the scope of the subject invention are polynucleotides which have the same nucleotide sequences of a polynucleotide exemplified herein except for nucleotide substitutions, additions, or deletions within the sequence of the polynucleotide, as long as these variant polynucleotides retain substantially the same relevant functional activity as the polynucleotides specifically exemplified herein (e.g., they encode a protein having the same amino acid sequence or the same functional activity as encoded by the exemplified polynucleotide). Thus, the polynucleotides disclosed herein should be understood to include variants and fragments, as discussed above, of the specifically exemplified sequences.

The subject invention also contemplates those polynucleotide molecules having sequences which are sufficiently homologous with the polynucleotide sequences of the invention so as to permit hybridization with that sequence under standard stringent conditions and standard methods (Maniatis, T. et al, 1982). Polynucleotides described herein can also be defined in terms of more particular identity and/or similarity ranges with those exemplified herein. The sequence identity will typically be greater than 60%, preferably greater than 75%, more preferably greater than 80%, even more preferably greater than 90%, and can be greater than 95%. The identity and/or similarity of a sequence can be 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91 , 92, 93, 94, 95, 96, 97, 98, or 99% or greater as compared to a sequence exemplified herein. Unless otherwise specified, as used herein percent sequence identity and/or similarity of two sequences can be determined using the algorithm of Karlin and Altschul (1990), modified as in Karlin and Altschul (1993). Such an algorithm is incorporated into the NBLAST and XBLAST programs of Altschul et al. (1990). BLAST searches can be performed with the NBLAST program, score = 100, wordlength = 12, to obtain sequences with the desired percent sequence identity. To obtain gapped alignments for comparison purposes, Gapped BLAST can be used as described in Altschul et al. (1997). When utilizing BLAST and Gapped BLAST programs, the default parameters of the respective programs (NBLAST and XBLAST) can be used. See NCBI/N1H website.

The present invention also concerns pharmaceutical compositions comprising the vector system or the viral vector system or the host cells of the invention optionally in combination with a pharmaceutically acceptable carrier, diluent, excipient or adjuvant. The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s), and other carrier agents that may aid or increase the viral entry into the target site (such as for example a lipid delivery system). The construct or vector can be administered in vivo or ex vivo.

Pharmaceutical compositions adapted for topical or parenteral administration, comprising an amount of a compound, constitute a preferred embodiment of the invention. For parenteral administration, the compositions may be best used in the form of a sterile aqueous solution which may contain other substances, for example enough salts or monosaccharides to make the solution isotonic with blood. The pharmaceutical composition of the present invention may be delivered to the retina preferentially via the subretinal injection or it can also be prepared in the form of injectable suspension, eye lotion or ophthalmic ointment that can be delivered to the retina with a non-invasive procedure.

The dose administered to a patient, particularly a human, in the context of the present invention should be sufficient to achieve a therapeutic response in the patient over a reasonable time frame, without lethal toxicity, and preferably causing no more than an acceptable level of side effects or morbidity. One skilled in the art will recognize that dosage will depend upon a variety of factors including the condition (health) of the subject, the body weight of the subject, kind of concurrent treatment, if any, frequency of treatment, therapeutic ratio, as well as the severity and stage of the pathological condition.

The methods of the present invention can be used with humans and other animals. As used herein, the terms "patient" and "subject" are used interchangeably and are intended to include such human and non-human species. Likewise, in vitro methods of the present invention can be earned out on cells of such human and non- human species.

The subject invention also concerns kits comprising the construct system or viral vector system or the host cells of the invention in one or more containers. Kits of the invention can optionally include pharmaceutically acceptable carriers and/or diluents. In one embodiment, a kit of the invention includes one or more other components, adjuncts, or adjuvants as described herein. In one embodiment, a kit of the invention includes instructions or packaging materials that describe how to administer a vector system of the kit. Containers of the kit can be of any suitable material, e.g., glass, plastic, metal, etc., and of any suitable size, shape, or configuration. In one embodiment, the construct system or viral vector system or the host cells of the invention is provided in the kit as a solid. In another embodiment, the construct system or viral vector system or the host cells of the invention is provided in the kit as a liquid or solution. In one embodiment, the kit comprises an ampoule or syringe containing the construct system or viral vector system or the host cells of the invention in liquid or solution form.

The present invention also provides a pharmaceutical composition for treating an individual by gene therapy, wherein the composition comprises a therapeutically effective amount of the vector system or viral vector system or host cell of the present invention comprising one or more deliverable therapeutic and/or diagnostic transgenes(s) or a viral particle produced by or obtained from same. The pharmaceutical composition may be for human or animal usage. Typically, an ordinary skilled clinician will determine the actual dosage which will be most suitable for an individual subject and it will vary with the age, weight and response of the particular individual and administration route. A dose range between 1x10e10 and 1x10e15 genome copies of each vector/kg, preferentially between 1x10e11 and 1x10e13 genome copies of each vector/kg are expected to be effective in humans. A dose range between 1x10e10 and 1x10e15 genome copies of each vector/eye, preferentially between 1x10e10 and 1x10e13 are expected to be effective for ocular administration.

Dosage regimes and effective amounts to be administered can be determined by ordinarily skilled clinicians. Administration may be in the form of a single dose or multiple doses. General methods for performing gene therapy using polynucleotides, expression constructs, and vectors are known in the art (see, for example, Gene Therapy: Principles and Applications, Springer Verlag 1999; and U.S. Patent Nos. 6,461 ,606; 6,204,251 and 6,106,826). The subject invention also concerns methods for expressing a selected polypeptide in a cell. In one embodiment, the method comprises incorporating in the cell the vector system of the invention that comprises polynucleotide sequences encoding the selected polypeptide and expressing the polynucleotide sequences in the cell. The selected polypeptide can be one that is heterologous to the cell. In one embodiment, the cell is a mammalian cell. In one embodiment, the cell is a human cell. In one embodiment, the cell is a photoreceptor cell or an RPE cell. The cell may also be a muscle cell, in particular a skeletal muscle cell, a lung cell, a pancreas cell, a liver cell, a kidney cell, an intestine cell, a blood cell. In a specific embodiment, the cell is a cone cell or a rod cell. In a specific embodiment, the cell is a human cone cell or rod cell.

### SEQUENCES

**AP1** (SEQ ID No. 24)
AP2 (SEQ ID No. 25)
AK **seqA** (SEQ ID No. 22)
AK seqB (SEQ ID No. 23)
**AP** (SEQ ID No. 26)
**Left ITR2** (SEQ ID No. 29)
**Right ITR2** (SEQ ID No. 30)
**Left ITR5** (SEQ ID No. 31)
**Right ITR5** (SEQ ID No. 32)
**CMV**
***CMV enhancer*** (SEQ ID No. 33)
***CMV promoter*** (SEQ ID No. 34)
***Chimeric intron (SV40 intron)*** (SEQ ID No. 35)
**hGRK1 promoter** (SEQ ID No. 36)
**CBA hybrid promoter**
*CMV enhancer* (SEQ ID No. 37)
*CBA promoter* (SEQ ID No. 38)
**IRBP** (SEQ ID No. 39)
**Splicing donor signal** (SEQ ID No. 27)
**miR-let 7b degradation signal** (SEQ ID No. 40)
**4xmiR-let 7b degradation signal** (SEQ ID No. 41)
**miR-26a degradation signal** (SEQ ID No. 13)
**4xmiR-26a degradation signal** (SEQ ID No. 18)
**miR-204 degradation signal** (SEQ ID No. 11)
**miR-124 degradation signal** (SEQ ID No. 12)
**3xmiR-204+3xmiR-124 degradation signal** (SEQ ID No. 17)
**CL1 degradation signal (degron)**
Nucleotidic sequence: (SEQ ID No. 16)
Aminoacidic sequence: (SEQ ID No. 1)
**CL2 degradation signal (degron)**
Nucleotidic sequence: (SEQ ID No. 42)
Aminoacidic sequence: (SEQ ID No. 2)
**CL6 degradation signal (degron)**
Nucleotidic sequence: (SEQ ID No. 43)
Aminoacidic sequence: (SEQ ID No. 3)
**CL9 degradation signal (degron)**
Nucleotidic sequence: (SEQ ID No. 44)
Aminoacidic sequence: (SEQ ID No. 4)
**CL10 degradation signal (degron)**
Nucleotidic sequence: (SEQ ID No. 45)
Aminoacidic sequence: (SEQ ID No. 5)
**CL11 degradation signal (degron)**
Nucleotidic sequence: (SEQ ID No. 46)
Aminoacidic sequence: (SEQ ID No. 6)
**CL12 degradation signal (degron)**
Nucleotidic sequence: (SEQ ID No. 47)
Aminoacidic sequence: (SEQ ID No. 7)
**CL15 degradation signal (degron)**
Nucleotidic sequence: (SEQ ID No. 48)
Aminoacidic sequence: (SEQ ID No. 8)
**CL16 degradation signal (degron)**
Nucleotidic sequence: (SEQ ID No. 49)
Aminoacidic sequence: (SEQ ID No. 9)
**SL17 degradation signal (degron)**
Nucleotidic sequence: (SEQ ID No. 50)
Aminoacidic sequence: (SEQ ID No. 10)
**PB29 degradation signal (degron)**
Nucleotidic sequence: (SEQ ID No. 19)
Aminoacidic sequence: (SEQ ID No. 15)
**Short PB29 degradation signal (degron)**
Nucleotidic sequence: (SEQ ID No. 20)
Aminoacidic sequence: (SEQ ID No. 14)
**3x PB29 degradation signal (degron)** (SEQ ID No. 21)
**Artificial Stop codons** (SEQ ID No. 51)
**Splicing acceptor signal** (SEQ ID No. 28)
**SV40 Poly** A (SEQ ID No. 52)
**ABCA4 5'** (SEQ ID No. 53)
**hGRK1-5' ABCA4+AK+CL1 Full length sequence** (SEQ ID No. 54) 
CTGCGCGCTCGCTCGCTCACTGAGGCCGCCCGGGCAAAGCCCGGGCGTCGGGCGACCTTTGGTCGCCC
GGCCTCAGTGAGCGAGCGAGCGCGCAGAGAGGGAGTGGCCAACTCCATCACTAGGGGTTCCTtgtagt
taatgattaacccgccatgctacttatctacgtagccatgctctaggaagatcttcaatattggccattagccatattattcattggttatat
agcataaatcaatattggctattggccattgcatacgttgtatctatatcataatatgtacatttatattggctcatgtccaatatgaccgcc
atgttggcattgattattgactagtgggccccagaagcctggtggttgtttgtccttctcaggggaaaagtgaggcggccccttgg
aggaaggggccgggcagaatgatctaatcggattccaagcagctcaggggattgtctttttctagcaccttcttgccactcctaa
gcgtcctccgtgaccccggctgggatttagcctggtgctgtgtcagccccgggctcccaggggcttcccagtggtccccaggaacc
ctcgacagggccagggcgtctctctcgtccagcaagggcagggacgggccacaggcaagggcgcggccgccatgggcttcgtg
agacagatacagcttttgctctggaagaactggaccctgcggaaaaggcaaaagattcgctttgtggtggaactcgtgtggcctttatct
ttatttctggtcttgatctggttaaggaatgccaacccgctctacagccatcatgaatgccatttccccaacaaggcgatgccctcagcag
gaatgctgccgtggctccaggggatcttctgcaatgtgaacaatccctgttttcaaagccccaccccaggagaatctcctggaattgtgtc
aaactataacaactccatcttggcaagggtatatcgagattttcaagaactcctcatgaatgcaccagagagccagcaccttggccgtat
ttggacagagctacacatcttgtcccaattcatggacaccctccggactcacccggagagaattgcaggaagaggaattcgaataagg
gatatcttgaaagatgaagaaacactgacactatttctcattaaaaacatcggcctgtctgactcagtggtctaccttctgatcaactctc
aagtccgtccagagcagttcgctcatggagtcccggacctggcgctgaaggacatcgcctgcagcgaggccctcctggagcgcttcatc
atcttcagccagagacgcggggcaaagacggtgcgctatgccctgtgctccctctcccagggcaccctacagtggatagaagacactct
gtatgccaacgtggacttcttcaagctcttccgtgtgcttcccacactcctagacagccgttctcaaggtatcaatctgagatcttgggggag
gaatattatctgatatgtcaccaagaattcaagagtttatccatcggccgagtatgcaggacttgctgtgggtgaccaggcccctcatgc
agaatggtggtccagagacctttacaaagctgatgggcatcctgtctgacctcctgtgtggctaccccgagggaggtggctctcgggtgc
tctccttcaactggtatgaagacaataactataaggcctttctggggattgactccacaaggaaggatcctatctattcttatgacagaag
aacaacatccttttgtaatgcattgatccagagcctggagtcaaatcctttaaccaaaatcgcttggagggcggcaaagcctttgctgat
gggaaaaatcctgtacactcctgattcacctgcagcacgaaggatactgaagaatgccaactcaacttttgaagaactggaacacgtta
ggaagttggtcaaagcctgggaagaagtagggccccagatctggtacttctttgacaacagcacacagatgaacatgatcagagatac
cctggggaacccaacagtaaaagactttttgaataggcagcttggtgaagaaggtattactgctgaagccatcctaaacttcctctacaa
gggccctcgggaaagccaggctgacgacatggccaacttcgactggagggacatatttaacatcactgatcgcaccctccgccttgtca
atcaatacctggagtgcttggtcctggataagtttgaaagctacaatgatgaaactcagctcacccaacgtgccctctctctactggagg
aaaacatgttctgggccggagtggtattccctgacatgtatccctggaccagctctctaccaccccacgtgaagtataagatccgaatgg
acatagacgtggtggagaaaaccaataagattaaagacaggtattgggattctggtcccagagctgatcccgtggaagatttccggtac
atctggggcgggtttgcctatctgcaggacatggttgaacaggggatcacaaggagccaggtgcaggcggaggctccagttggaatct
acctccagcagatgccctacccctgcttcgtggacgattctttcatgatcatcctgaaccgctgtttccctatcttcatggtgctggcatgga
tctactctgtctccatgactgtgaagagcatcgtcttggagaaggagttgcgactgaaggagaccttgaaaaatcagggtgtctccaatg
 
cagtgatttggtgtacctggttcctggacagcttctccatcatgtcgatgagcatcttcctcctgacgatattcatcatgcatggaagaatc
ctacattacagcgacccattcatcctcttcctgttcttgttggctttctccactgccaccatcatgctgtgctttctgctcagcaccttcttctc
caaggccagtctggcagcagcctgtagtggtgtcatctatttcaccctctacctgccacacatcctgtgcttcgcctggcaggaccgcatg
accgctgagctgaagaaggctgtgagcttactgtctccggtggcatttggatttggcactgagtacctggttcgctttgaagagcaaggc
ctggggctgcagtggagcaacatcgggaacagtcccacggaaggggacgaattcagcttcctgctgtccatgcagatgatgctccttga
tgctgctgtctatggcttactcgcttggtaccttgatcaggtgtttccaggagactatggaaccccacttccttggtactttcttctacaaga
gtcgtattggcttggcggtgaagggtgttcaaccagagaagaaagagccctggaaaagaccgagcccctaacagaggaaacggagg
atccagagcacccagaaggaatacacgactccttctttgaacgtgagcatccagggtgggttcctggggtatgcgtgaagaatctggta
aagatttttgagccctgtggccggccagctgtggaccgtctgaacatcaccttctacgagaaccagatcaccgcattcctgggccacaat
ggagctgggaaaaccaccaccttgtaagtatcaaggttacaagacaggtttaaggagaccaatagaaactgggcttgtcgagacag
agaagactcttgcgtttctGGGATTTTCCGATTTCGGCCTATTGGTTAAAAAATGAGCTGATTTAACAAAAAT
TTAACGCGAATTTTAACAAAATattaacgtttataatttcaggtggcatctttcccgcctgcaagaactggttcagcagcctga
gccacttcgtgatccacctgcaattgAGGAACCCCTAGTGATGGAGTTGGCCACTCCCTCTCTGCGCGCTCGCTC
GCTCACTGAGGCCGGGCGACCAAAGGTCGCCCGACGCCCGGGCTTTGCCCGGGCGGCCTCAGTGAGC
GAGCGAGCGCGCAG
 Legend:
   ITR: uppercases bold
   hGRK promoter: lowercases bold italic
   ABCA4 5': lowercase underlined
   SDS: lowercase bold
   AK: uppercase
   CL1: lowercase italic underlined
**Abca4_3'** (SEQ ID No. 55)
**ABCA4 3'+AK_SV40 Full length sequence** (SEQ ID No. 56) 
CTGCGCGCTCGCTCGCTCACTGAGGCCGCCCGGGCAAAGCCCGGGCGTCGGGCGACCTTTGGTCGCCC
GGCCTCAGTGAGCGAGCGAGCGCGCAGAGAGGGAGTGGCCAACTCCATCACTAGGGGTTCCTggatcc
GGGATTTTCCGATTTCGGCCTATTGGTTAAAAAATGAGCTGATTTAACAAAAATTTAACGCGAATTTTA
ACAAAATattaacgtttataatttcaggtggcatctttcgataggcacctattggtcttactgacatccactttgcctttctctccacagg
tccatcctgacgggtctgttgccaccaacctctgggactgtgctcgttgggggaagggacattgaaaccagcctggatgcagtccggcag
agccttggcatgtgtccacagcacaacatcctgttccaccacctcacggtggctgagcacatgctgttctatgcccagctgaaaggaaag
tcccaggaggaggcccagctggagatggaagccatgttggaggacacaggcctccaccacaagcggaatgaagaggctcaggaccta
tcaggtggcatgcagagaaagctgtcggttgccattgcctttgtgggagatgccaaggtggtgattctggacgaacccacctctggggtg
gacccttactcgagacgctcaatctgggatctgctcctgaagtatcgctcaggcagaaccatcatcatgtccactcaccacatggacgag
gccgacctccttggggaccgcattgccatcattgcccagggaaggctctactgctcaggcaccccactcttcctgaagaactgctttggca
caggcttgtacttaaccttggtgcgcaagatgaaaaacatccagagccaaaggaaaggcagtgaggggacctgcagctgctcgtctaa
gggtttctccaccacgtgtccagcccacgtcgatgacctaactccagaacaagtcctggatggggatgtaaatgagctgatggatgtagt
tctccaccatgttccagaggcaaagctggtggagtgcattggtcaagaacttatcttccttcttccaaataagaacttcaagcacagagc
atatgccagccttttcagagagctggaggagacgctggctgaccttggtctcagcagttttggaatttctgacactcccctggaagagatt
 
tttctgaaggtcacggaggattctgattcaggacctctgtttgcgggtggcgctcagcagaaaagagaaacgtcaaccccgacaccc
ctgcttgggtcccagagagaaggctggacagacaccccaggactccaatgtctgctccccaggggcgccggctgctcacccagagggc
cagcctcccccagagccagagtgcccaggcccgcagctcaacacggggacacagctggtcctccagcatgtgcaggcgctgctggtca
agagattccaacacaccatccgcagccacaaggacttcctggcgcagatcgtgctcccggctacctttgtgtttttggctctgatgctttct
attgttatccctccttttggcgaataccccgctttgacccttcacccctggatatatgggcagcagtacaccttcttcagcatggatgaacc
aggcagtgagcagttcacggtacttgcagacgtcctcctgaataagccaggctttggcaaccgctgcctgaaggaagggtggcttccgg
agtacccctgtggcaactcaacaccctggaagactccttctgtgtccccaaacatcacccagctgttccagaagcagaaatggacacag
gtcaacccttcaccatcctgcaggtgcagcaccagggagaagctcaccatgctgccagagtgccccgagggtgccggggcctcccgc
ccccccagagaacacagcgcagcacggaaattctacaagacctgacggacaggaacatctccgacttcttggtaaaaacgtatcctgc
tcttataagaagcagcttaaagagcaaattctgggtcaatgaacagaggtatggaggaatttccattggaggaaagctcccagtcgtcc
ccatcacgggggaagcacttgttgggtttttaagcgaccttggccggatcatgaatgtgagcgggggccctatcactagagaggcctcta
aagaaatacctgatttccttaaacatctagaaactgaagacaacattaaggtgtggtttaataacaaaggctggcatgccctggtcagct
ttctcaatgtggcccacaacgccatcttacgggccagcctgcctaaggacagaagccccgaggagtatggaatcaccgtcattagccaa
cccctgaacctgaccaaggagcagctctcagagattacagtgctgaccacttcagtggatgctgtggttgccatctgcgtgattttctcca
tgtccttcgtcccagccagctttgtcctttatttgatccaggagcgggtgaacaaatccaagcacctccagtttatcagtggagtgagccc
caccacctactgggtaaccaacttcctctgggacatcatgaattattccgtgagtgctgggctggtggtgggcatcttcatcgggtttcag
aagaaagcctacacttctccagaaaaccttcctgcccttgtggcactgctcctgctgtatggatgggcggtcattcccatgatgtacccag
catccttcctgtttgatgtccccagcacagcctatgtggctttatcttgtgctaatctgttcatcggcatcaacagcagtgctattaccttcat
cttggaattatttgagaataaccggacgctgctcaggttcaacgccgtgctgaggaagctgctcattgtcttcccccacttctgcctgggc
cggggcctcattgaccttgcactgagccaggctgtgacagatgtctatgcccggtttggtgaggagcactctgcaaatccgttccactgg
gacctgattgggaagaacctgtttgccatggtggtggaaggggtggtgtacttcctcctgaccctgctggtccagcgccacttcttcctctc
ccaatggattgccgagcccactaaggagcccattgttgatgaagatgatgatgtggctgaagaaagacaaagaattattactggtgga
aataaaactgacatcttaaggctacatgaactaaccaagatttatccaggcacctccagcccagcagtggacaggctgtgtgtcggagt
tcgccctggagagtgctttggcctcctgggagtgaatggtgccggcaaaacaaccacattcaagatgctcactggggacaccacagtga
cctcaggggatgccaccgtagcaggcaagagtattttaaccaatatttctgaagtccatcaaaatatgggctactgtcctcagtttgatgc
aatcgatgagctgctcacaggacgagaacatctttacctttatgcccggcttcgaggtgtaccagcagaagaaatcgaaaaggttgcaa
actggagtattaagagcctgggcctgactgtctacgccgactgcctggctggcacgtacagtgggggcaacaagcggaaactctccaca
gccatcgcactcattggctgcccaccgctggtgctgctggatgagcccaccacagggatggacccccaggcacgccgcatgctgtggaa
cgtcatcgtgagcatcatcagagaagggagggctgtggtcctcacatcccacagcatggaagaatgtgaggcactgtgtacccggctgg
ccatcatggtaaagggcgcctttcgatgtatgggcaccattcagcatctcaagtccaaatttggagatggctatatcgtcacaatgaaga
tcaaatccccgaaggacgacctgcttcctgacctgaaccctgtggagcagttcttccaggggaacttcccaggcagtgtgcagagggag
aggcactacaacatgctccagttccaggtctcctcctcctccctggcgaggatcttccagctcctcctctcccacaaggacagcctgctca
tcgaggagtactcagtcacacagaccacactggaccaggtgtttgtaaattttgctaaacagcagactgaaagtcatgacctccctctgc
accctcgagctgctggagccagtcgacaagcccaggactgagcggccgcttcgagcagacatgataagatacattgatgagtttgg
acaaaccacaactagaatgcagtgaaaaaaatgctttatttgtgaaatttgtgatgctattgctttatttgtaaccattataagct
gcaataaacaagttaacaacaacaattgcattcattttatgtttcaggttcagggggagatgtgggaggttttttaaagcaagt
aaaacctctacaaatgtggtaaaatcgataaggatcttcctagagcatggctacgtagataagtagcatggcgggttaatcattaac
tacaAGGAACCCCTAGTGATGGAGTTGGCCACTCCCTCTCTGCGCGCTCGCTCGCTCACTGAGGCCGGG
CGACCAAAGGTCGCCCGACGCCCGGGCTTTGCCCGGGCGGCCTCAGTGAGCGAGCGAGCGCGCAG
 Legend:
   ITR: uppercases bold underlined
   AK: uppercase
   SAS: lowercase bold
   ABCA4 3': lowercase underlined
   SV40 polyA: lowercases bold italic
**CMV 5' ABCA4-SD-AK Full length sequence** (SEQ ID No. 57)
**AK-SA-3' ABCA4-3XFLAG-SV40 Full length sequence** (SEQ ID No. 58).
**CMV 5' ABCA4-SD-AP1 Full length sequence** (SEQ ID No. 59)
**AP1-SA-3' ABCA4-3XFLAG-SV40 Full length sequence** (SEQ ID No. 60)
**CMV 5' ABCA4-SD-AP2 Full length sequence** (SEQ ID No. 61)
**AP2-SA-3' ABCA4-3XFLAG-SV40 Full length sequence** (SEQ ID No. 62)
**CMV 5' ABCA4-SD-AP Full length sequence** (SEQ ID No. 63)
**AP-SA-3' ABCA4-3XFLAG-SV40 Full length sequence** (SEQ ID No. 64)
**hGRK1 5' ABCA4-SD-AP1 Full length sequence** (SEQ ID No. 65)
**GRK1 5' ABCA4-SD-AP2 Full length sequence** (SEQ ID No. 66)
**ITR5-CMV 5' ABCA4-SD-AK-ITR2 Full length sequence** (SEQ ID No. 67)
**ITR2-AK-SA-3' ABCA4-SV40-ITR5 Full length sequence** (SEQ ID No. 68)
**ITR5-CBA 5' MYO7A-SD-AK-ITR2 Full length sequence** (SEQ ID No. 69)
**ITR2-AK-SA-3' MYO7A-HA-BGH-ITR5 Full length sequence** (SEQ ID No. 70)
**CMV 5' ABCA4-3XFLAG-SD-AK-4xmiR26a Full length sequence** (SEQ ID No. 71)
**CMV** 5' **ABCA4-3XFLAG-SD-AK-3xmiR204+3xmir124 Full length sequence** (SEQ ID No.72)
**CMV 5' ABCA4-3XFLAG-SD-AK-CL1 Full length sequence** (SEQ ID No. 73)
**AK-STOP-SA-3' ABCA4-3XFLAG-SV40 Full length sequence** (SEQ ID No. 74)
**AK-PB29-SA-3' ABCA4-3XFLAG-SV40 Full length sequence** (SEQ ID No. 75)
**AK-3XPB29-SA-3' ABCA4-3XFLAG-SV40 Full length sequence** (SEQ ID No. 76)
**AK-UBIQUITIN-SA-3' ABCA4-3XFLAG-SV40 Full length sequence** (SEQ ID No. 77).

The present invention will now be illustrated by means of non-limiting examples in reference to the following drawings.

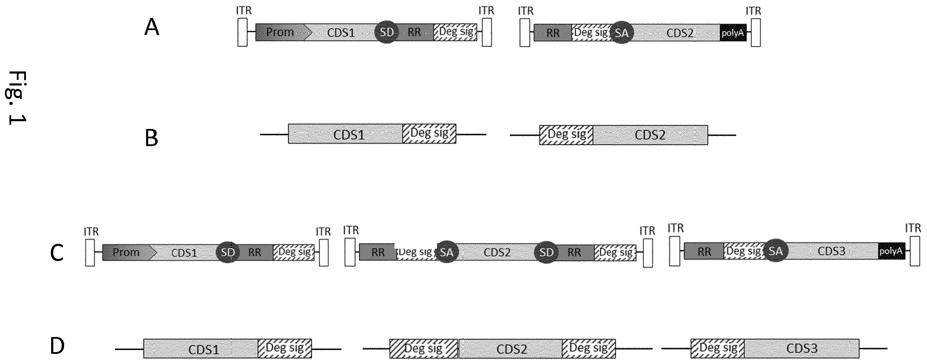

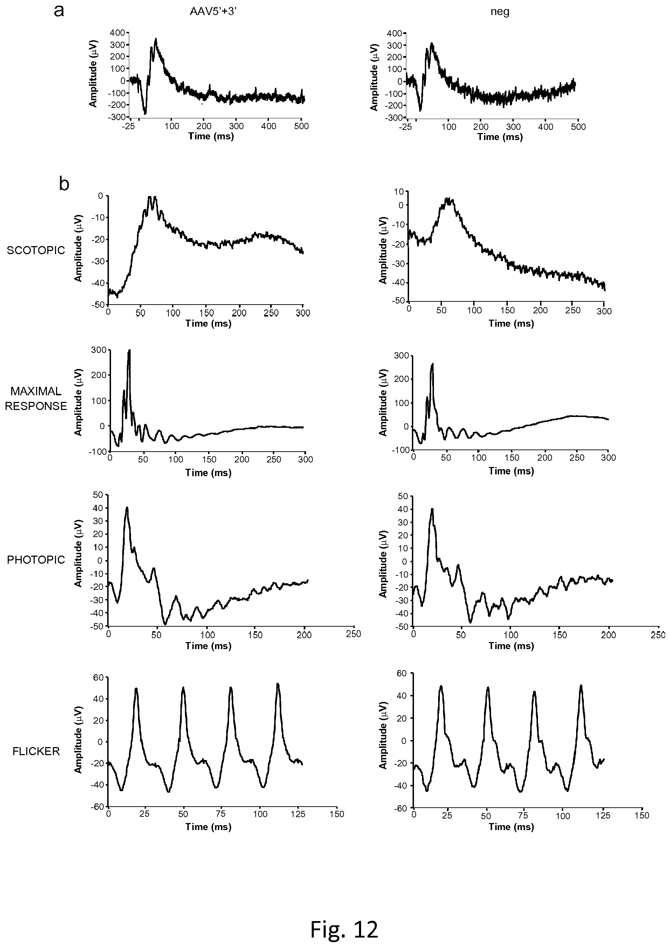

*Figure 1**. Schematic representation of multiple-vector strategies of present invention examples.* ITR: inverted terminal repeats; Prom: promoter; CDS, coding sequence; SD, splicing donor signal; RR: recombinogenic regions, AK or from alkaline phosphatase (API, AP2 and AP); Deg Sig; degradation signals (see Table 2); SA, splicing acceptor signal; pA, polyadenylation signal. A and C: (dual or triple) hybrid vectors strategy, including transplicing and recombinogenic regions, according to a preferred embodiment of the invention B and D: (dual or triple) vectors overlapping vectors strategy. For additional examples, see Figs. 12-14.

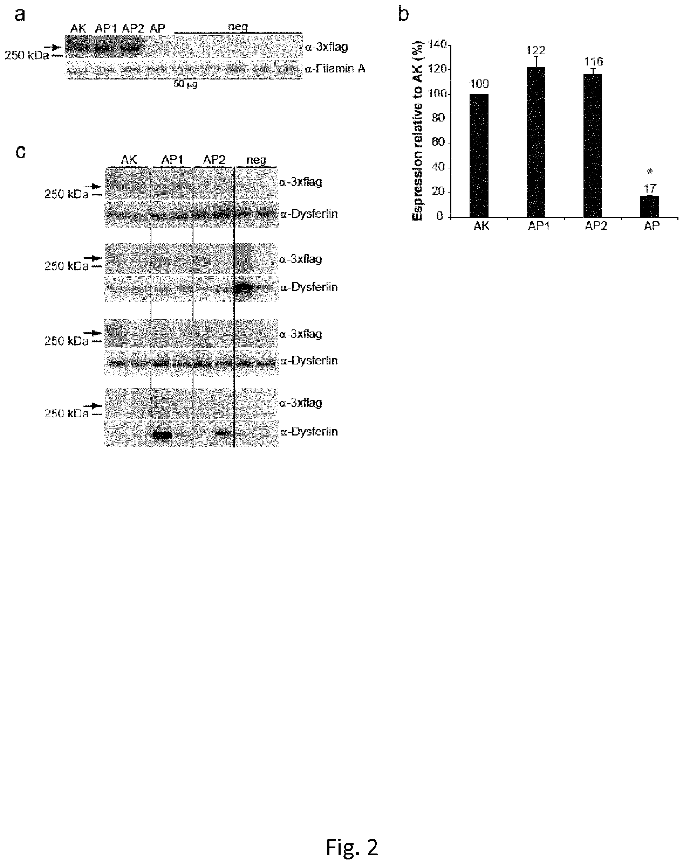

*Figure 2**. Efficient ABCA4 protein expression using the AK, AP1 and AP2 regions of homology* (a, c) Representative Western blot analysis of (a) HEK293 cells (50 micrograms/lane) infected with dual AAV2/2 (AAV serotype 2, with homologous ITR from AAV2) vectors or (c) C57BL/6 retinas (whole retinal lysates) injected with dual AAV2/8 (AAV serotype 8, with homologous ITR from AAV2) vectors encoding for *ABCA4.* The arrows indicate full-length proteins, the molecular weight ladder is depicted on the left. (b) Quantification of ABCA4 protein bands from Western blot analysis in (a). The intensity of the ABCA4 bands in (a) was divided by the intensity of the Filamin A bands. The histograms show the expression of proteins as a percentage relative to dual AAV hybrid AK vectors, the mean value is depicted above the corresponding bar. Values are represented as: mean ± s.e.m. (standard error of the mean). ^{∗}pANOVA < 0.05; the asterisk indicate significant differences with AK, API and AP2. (a-c) AK: cells infected or eyes injected with dual AAV hybrid AK vectors; API: cells infected or eyes injected with dual AAV hybrid API vectors; AP2: cells infected or eyes injected with dual AAV hybrid AP2 vectors; AP: cells infected with dual AAV hybrid AP vectors; neg: cells infected or eyes injected with either the 3'-half vectors or EGFP expressing vectors, as negative controls. α-3xflag: Western blot with anti-3xflag antibodies; α-Filamin A, Western blot with anti-Filamin A antibodies, used as loading control; α-Dysferlin, Western blot with anti-Dysferlin antibodies, used as loading control.

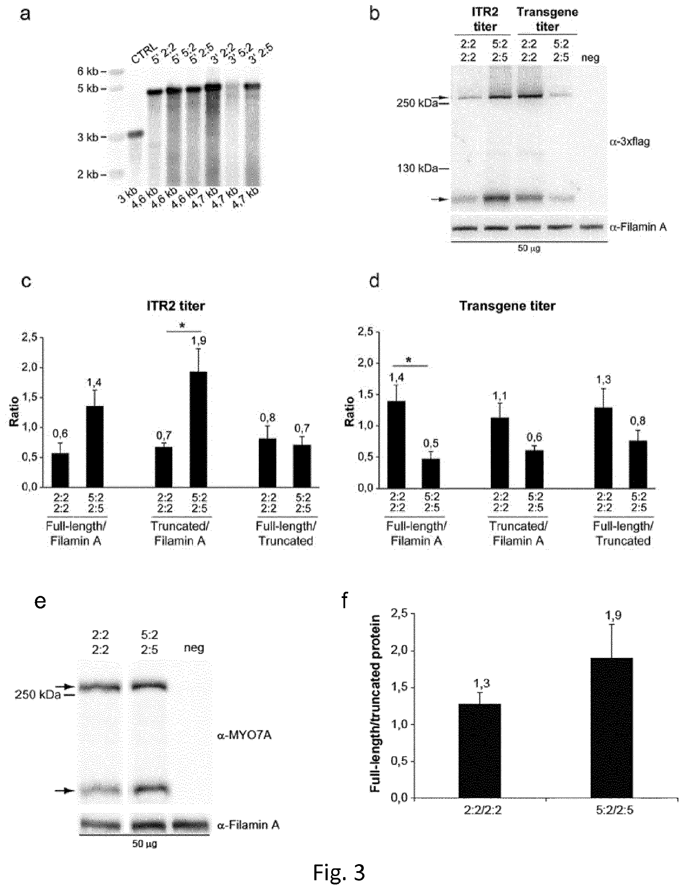

*Figure 3**. Genome and transduction efficiency of vectors with heterologous ITR2 and ITR5.* (a) Alkaline Southern blot analysis of DNA extracted from 3 × 1010 GC of both 5'- and 3'-ABCA4-half vectors with either homologous (2:2) or heterologous (5:2 or 2:5) ITR, and of a control AAV preparation with homologous ITR2 (CTRL). The expected size of each genome is depicted beloweach lane. The molecular weight marker (kb) is depicted on the left 5': 5'-half vector; 3': 3'-half vector. (b-d) Representative Western blot analysis and quantification of HEK293 cells infected with dual AAV2/2 hybrid ABCA4 vectors with either heterologous ITR2 and ITR5 or homologous ITR2 at m.o.i. based on either the ITR2 (b and c) or the transgene (b and d) titre. The Western blot images (b) are representative of n = 3 independent experiments; the quantifications (c and d) are from n = 3 independent experiments. (b) The upper arrow indicates full-length ABCA4 protein, the lower arrow indicates truncated proteins; the molecular weight ladder is depicted on the left. The micrograms of proteins loaded are depicted below the image. α-3xflag: Western blot with anti-3×flag antibodies; α-Filamin A: Western blot with anti-Filamin A antibodies, used as loading control. (c and d) Quantification of full-length and truncated ABCA4 protein bands from Western blot analysis of cells infected with a dose of vector based on either the ITR2 (c) or the transgene (d) titre. The histograms show either the intensity of the full-length and truncated protein bands divided by that of the Filamin A bands or the intensity of the full-length protein bands divided by that of the truncated protein bands in the corresponding lane. Representative Western blot analysis and quantification of HEK293 cells infected with dual AAV2 (AAV serotype 2) hybrid vectors with either heterologous ITR2 and ITR5 or homologous ITR2 encoding for *MYO7A* (e, f). the Western blot images (e) are representative of and the quantifications (f) are from n=3 independent experiments. (e) The upper arrows indicate full-length proteins, the lower arrows indicate truncated proteins, the molecular weight ladder is depicted on the left. The micrograms of proteins loaded are depicted below the image. (f) Quantification of *MYO7A* protein bands from Western blot analysis.

The mean value is depicted above the corresponding bar. Values are represented as: mean ± s.e.m. ^{∗}p Student's t test ≤ 0.05.

2:2 2:2: cells infected with dual AAV hybrid vectors with homologous ITR from AAV2; 5:2 2:5: cells infected with dual AAV hybrid vectors with heterologous ITR from AAV2 and AAV5; neg: cells infected with EGFP-expressing vectors, as negative controls.

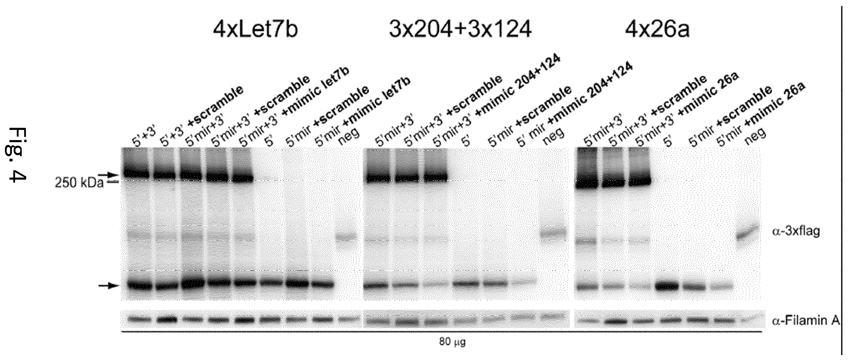

*Figure 4**. Inclusion of miR target sites in the 5'-half vectors does not result in significant reduction of truncated protein products*

Representative Western blot analysis of HEK293 cells infected with dual AAV2/2 (AAV serotype 2) hybrid vectors encoding for *ABCA4,* containing miR target sites for either miR-let7b (left panel), miR-204+124 (central panel) or miR-26a (right panel). The upper arrow indicates full-length ABCA4 proteins, the lower arrow indicates truncated proteins; the molecular weight ladder is depicted on the left. The micrograms of proteins loaded are depicted below the image. 5'+3': cells co-infected with 5'-half vectors without miR target sites and 3'-half vectors; 5'+3'+scrumble: cells co-infected with 5'-half vectors without miR target sites and 3'-half vectors in the presence of scramble miR mimics; 5'mir+3': cells co-infected with 5 '-half vectors containing miR target sites and 3'-half vectors; 5'mir+3'+ scramble: cells co-infected with 5'-half vectors containing miR target sites and 3'-half vectors in the presence of scramble miR mimics; 5'mir+3'+mimic let7b: cells co-infected with 5'-half vectors containing miR target sites and 3'-half vectors in the presence of mir-let7b mimics; 5' : cells infected with 5'-half vectors without miR target sites;5'mir: cells infected with 5'-half vectors containing miR target sites in the presence of scramble miR mimics; 5'mir+mimic let7b: cells infected with 5'-half vectors containing miR target sites in the presence of mir-let7b mimics; neg: control cells infected with either the 3'-half vectors or EGFP-expressing vectors; 5'mir+3'+mimic 204+124: cells co-infected with 5'-half vectors containing miR target sites and 3'-half vectors in the presence of mir-204 and 124 mimics; 5'mir+mimic 204+124: cells infected with 5'-half vectors containing miR target sites in the presence of mir-204 and 124 mimics; 5'mir+3'+mimic 26a: cells co-infected with 5'-half vectors containing miR target sites and 3'-half vectors in the presence of mir-26a mimics; 5'mir+mimic 26a: cells infected with 5'-half vectors containing miR target sites in the presence of mir-26a mimics. α-3xflag: Western blot with anti-3xflag antibodies; α-Filamin A, Western blot with anti-Filamin A antibodies, used as loading control

Scramble sequence correspond to sequence of a different miRNA, for instance in the experiment with mir-let7b mimics the scramble sequence was that of miR26a.

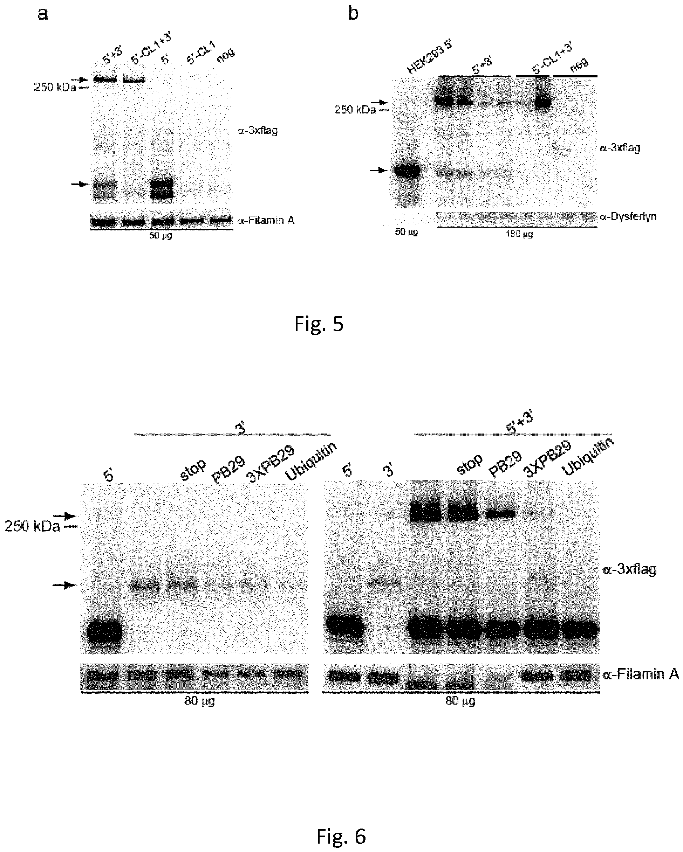

*Figure 5**. Inclusion of CL1 degradation signal in the 5'-half vectors results in significant reduction of truncated protein products*

Representative Western blot analysis of either (a) HEK293 cells infected with dual AAV2/2 (AAV serotype 2, with homologous ITR from AAV2) hybrid vectors or (b) pig eyes (RPE+retina) one month post-injection of dual AAV2/8 (AAV serotype 8, with homologous ITR from AAV2) hybrid vectors encoding for *ABCA4* and containing or not the CL1 degradation signal. The upper arrows indicate the full-length ABCA4 protein, the lower arrows indicate the truncated protein from the 5'-half vector; the molecular weight ladder is depicted on the left. The micrograms of proteins loaded are depicted below each image. 5'+3': cells co-infected or eyes co-injected with 5'-half vectors without CL1 and 3'-half vectors; **5'-CL1+3':** cells co-infected or eyes co-injected with 5'-half vectors containing CL1 and 3'-half vectors; 5': cells infected with 5'-half vectors wihtout CL1; **5'-CL1**: cells infected with 5'-half vectors containing CL1; neg: control cells infected or control eyes injected with either the 3'-half vectors or EGFP expressing vectors, as negative controls; α-3xflag: Western blot with anti-3xflag antibodies; **α-Filamin A**: Western blot with anti-Filamin A antibodies, used as loading control; **α-Dysferlin:** Western blot with anti-Dysferlin antibodies, used as loading control. (a) The Western blot image is representative of n = 3 independent experiments. (b) The Western blot image is representative of n= 5 eyes injected with 5'+3' vectors, n=2 eyes injected with 5'-CL1+3' vectors and n=5 of eyes injected with either the 3'-half vectors or EGFP expressing vectors as negative controls.

*Figure 6**. Inclusion of degradation signals in the 3'-half vectors results in slight reduction of truncated protein products*

Representative Western blot analysis of HEK293 cells infected with dual AAV2/2 hybrid vectors encoding for *ABCA4* and containing different degradation signals. The upper arrow indicates the full-length ABCA4 protein, the lower arrow indicates truncated protein products; the molecular weight ladder is depicted on the left. The micrograms of proteins loaded are depicted below each image. **5'+3':** cells co-infected with 5'- and 3'-half vectors without degradation signals; **5':** cells infected with 5'-half vectors; **3' (no label):** cells infected with 3'-half vectors without degradation signals; **stop:** cells infected with 3'-half vectors containing stop codons; **PB29:** cells infected with 3'-half vectors containing the PB29 degradation signal; **3xPB29:** cells infected with 3'-half vectors containing 3 tandem copies of the PB29 degradation signal; **Ubiquitin:** cells infected with 3'-half vectors containing the ubiquitin degradation signal. **α-3xflag:** Western blot with anti-3xflag antibodies; **α-Filamin A**: Western blot with anti-Filamin A antibodies, used as loading control.

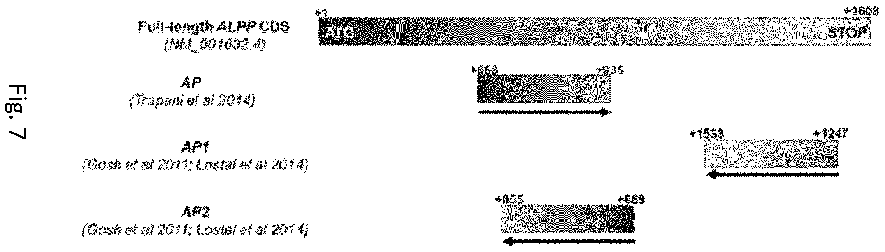

*Figure 7**: Schematic representation of the AP, AP1 and AP 2 regions of homology derived from ALPP (placental alkaline phosphatase) used in preferred embodiments of the present invention.* CDS: coding sequence

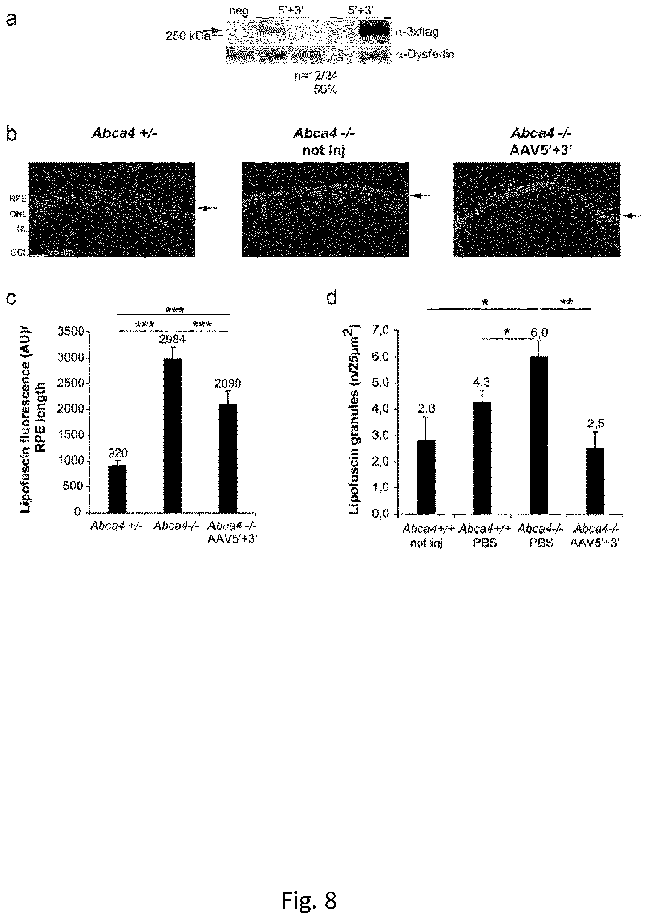

*Figure 8**: Subretinal delivery of improved dual AAV vectors results in ABCA4 expression in mouse photoreceptors and significant reduction of lipofuscin accumulation in the Abca4-*/*- mouse retina.* (a) Representative Western blot analysis of C57BL/6 retinas (whole retinal lysates) either injected with dual AAV2/8 hybrid ABCA4 vectors (5' ±3') or with negative controls (neg). The arrow indicates full-length proteins, the molecular weight ladder is depicted on the left. α-3xflag: Western blot with anti-3×flag antibodies; α-Dysferlin: Western blot with anti-Dysferlin antibodies, used as loading control. (b and c) Representative pictures (b) and quantification (c) of lipofuscin autofluorescence (red signal) in the retinas (RPE or RPE ±OS) of either pigmented Abca4+/- mice not injected or injected with AAV as control (Abca4+/-) or pigmented Abca4-/- mice either not injected (Abca4-/-) or injected with dual AAV hybrid ABCA4 vectors (Abca4-/- AAV5'+3'). (b) The scale bar (75 µm) is depicted in the picture. RPE: retinal pigment epithelium; ONL: outer nuclear layer; INL: inner nuclear layer; GCL: ganglion cell layer. The arrows indicate lipofuscin signal. (c) Mean lipofuscin autofluorescence in the temporal side of three sections for each sample. Mean autofluorescence in each section was normalized for the length of the underlying RPE. The mean value is depicted above the corresponding bar. Values are represented as mean ± s.e.m. ^{∗∗∗}p ANOVA < 0.0001. n = 4 eyes for each group. (d) Mean number of RPE lipofuscin granules counted in at least 40 fields (25 µm2)/retina of albino Abca4+/+ mice either not injected (Abca4+/+ not inj) or injected with PBS (Abca4+/+ PBS), and albino Abca4-/- mice injected with either PBS (Abca4-/- PBS) or dual AAV hybrid ABCA4 vectors (Abca4-/- AAV5'+3'). The mean value is depicted above the corresponding bar. Values are represented as mean ± s.e.m. ^{∗}pANOVA ≤ 0.05; ^{∗∗}pANOVA ≤0.01. n = 4 eyes from Abca4+/+ not inj; n = 4 eyes from Abca4+/+ PBS; n = 3 eyes from Abca4-/- PBS; n = 3 eyes from Abca4-/- AAV5'+ 3'.

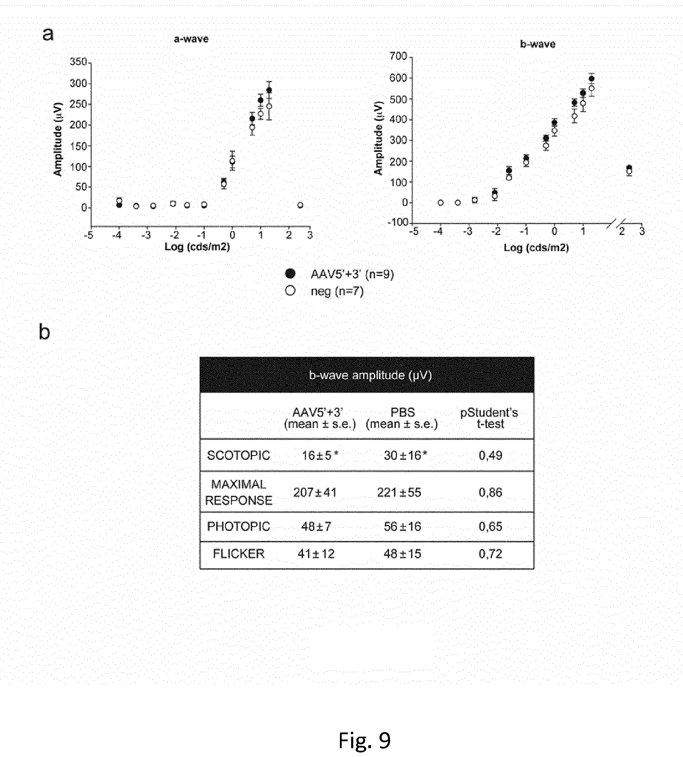

*Figure 9**: Similar electrical activity between either negative control or improved dual AAV-treated eyes of mice and pigs.* (a) Mean a-wave (left panel) and b-wave (right panel) amplitudes of C57BL/6 mice 1-month post-injection of either dualAAV hybrid ABCA4 vectors (AAV5'+ 3') or negative controls (i.e. negative control AAV vectors or PBS; neg). Data are presented as mean ± s.e.m.; n indicates the number of eyes analysed.

(b) Mean b-wave amplitudes (µV) in scotopic, maximal response, photopic and flicker ERG tests in pigs 1-month post-injection of either dual AAV hybrid ABCA4 vectors (AAV5'+ 3') or PBS. n = 5 eyes injected with dual AAV hybrid ABCA4 vectors; n = 4 injected with PBS; ^{∗}: n = 2.

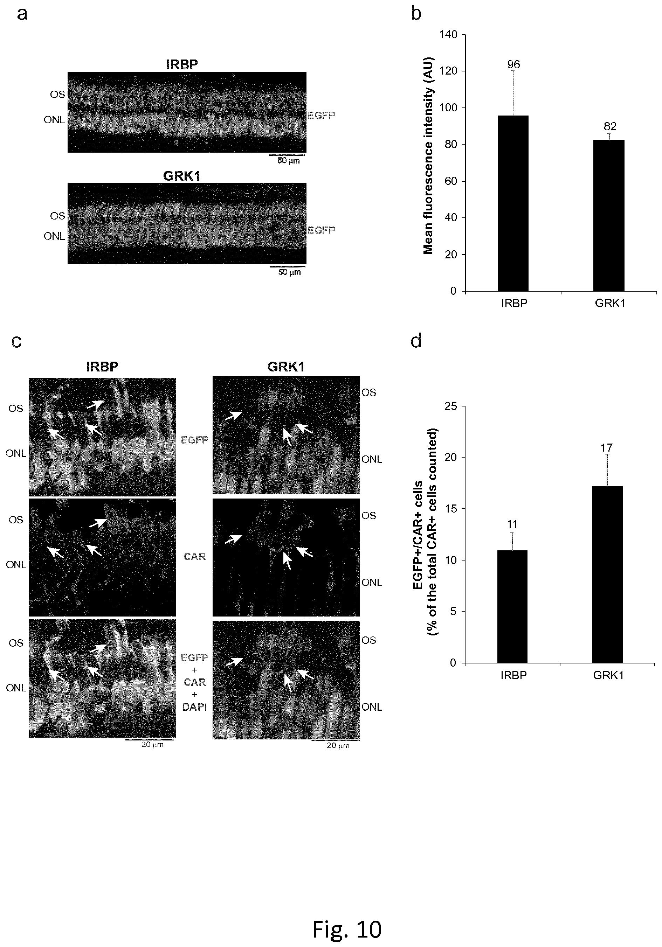

*Figure 10**: EGFP protein expression from the IRBP and GRK1 promoters in pig rod and cone photoreceptors.* Three month-old Large White pigs mice were injected subretinally with 1×10¹¹ GC/eye each of either AAV2/8-IRBP- or AAV2/8-GRK1-EGFP vectors. Retinal cryosections were obtained 4 weeks after injection and EGFP was analysed using fluorescence microscopy. (a-b) Representative images (a) and quantification (b) of fluorescence intensity in the PR layer. Fluorescence intensity was quantified for each group of animals on cryosections (six different fields/eye; 20x magnification), (c-d) Representative images (c) and quantification (d) of cone transduction efficiency. Cone transduction efficiency was evaluated on cryosections (six different fields/eye; 63x magnification) immunostained with an anti-LUMIf-hCAR antibody, and is expressed as number of cones expressing EGFP (EGFP+/CAR+) on total number of cones (CAR+) in each field. (a, c) The scale bar is depicted in the picture. (b-d) n=3 eyes injected with AAV2/8-IRBP-EGFP vectors; n=3 eyes injected with AAV2/8-GRK1-EGFP vectors. Values are represented as mean ± s.e.m. No significant differences were found using Student's t-test. OS: outer segments; ONL: outer nuclear layer; EGFP: native EGFP fluorescence; CAR: anti-cone arrestin staining; DAPI: 4',6'-diamidino-2-phenylindole staining. The arrows point at transduced cones.

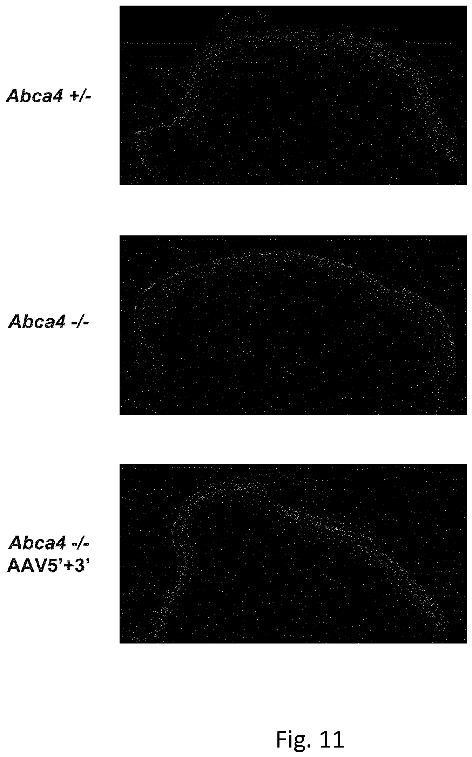

*Figure 11**: Subretinal delivery of improved dual AAV vectors results in significant reduction of lipofuscin accumulation in the Abca4-*/*- mouse retina.* Montage of images of the temporal (injected) side of retinal cross-sections showing lipofuscin auto fluorescence (red signal) in the retinas (RPE or RPE+OS) of either pigmented Abca4+/- mice not injected or injected with AAV as control (Abca4+/-) or pigmented Abca4-/- mice either not injected (Abca4-/-) or injected with dual AAV hybrid ABCA4 vectors (Abca4-/- AAV5'+3'). n= 4 eyes for each group. T: temporal side; N: nasal side.

*Figure 12**: Similar electrical activity between either negative control or improved dual AAV-treated eyes in mice and pigs.* (a) Representative ERG traces from C57BL/6 mice one month post-injection of either dual AAV hybrid ABCA4 vectors (AAV5'+3') or negative controls (i.e. negative control AAV vectors or PBS; neg). (b) Representative traces from scotopic, maximal response, photopic and flicker ERG tests in pigs one month post-injection of either dual AAV hybrid ABCA4 vectors (AAV5'+3') or PBS.

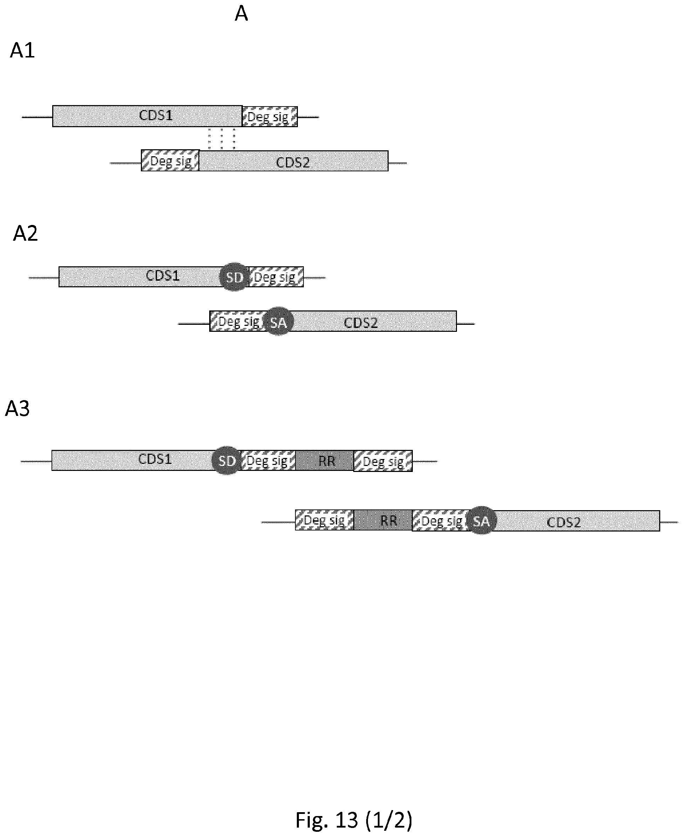

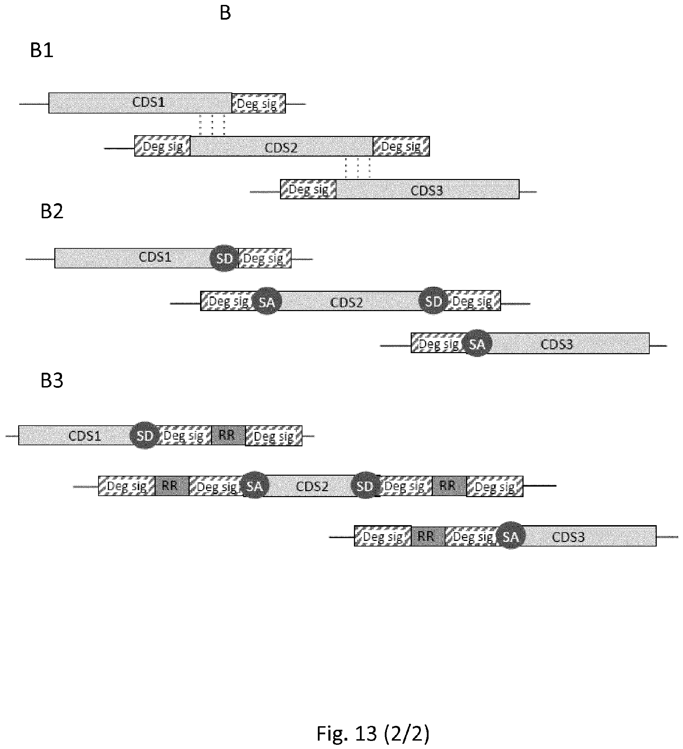

*Figure 13**. Schematic representation of vector system strategies, according to examples of the invention.* (A) Schematic representation of a vector system consisting of two vectors, according to preferred embodiments of the invention: a first vector comprises a first portion of the coding sequence (CDS1 portion), a second vector comprises a second portion (CDS2 portion) of the coding sequence. (A1) the reconstitution sequences of the vector system consist in the overlapping ends of the coding sequence portions. (A2) , the reconstitution sequences of the first and second vector consists respectively in a splicing donor and a splicing acceptor sequence. (A3) each reconstitution sequence comprises the splicing donor/acceptor, arranged as in A2 and it further comprises a recombinogenic region. A degradation signal is comprised in at least one of the vectors. The figure shows for each vector all the potential positions of the of the one or more degradation signals of the vector system, according to preferred non-limiting embodiments of the invention.

(B) Schematic representation of a vector system consisting of three vectors, according to preferred embodiments of the invention: a first vector comprises a first portion (CDS1 portion) of the coding sequence, a second vector comprises a second portion (CDS2 portion) of the coding sequence and a third vector comprises a third portion (CDS3 portion) of the coding sequence. (B1) the reconstitution sequences of the vector system consist in overlapping ends of the coding sequence portions (3' end of CDS1 overlapping with 5' end of CDS2; 3' end of CDS2 overlapping with 5' end of CDS3). (B2) the reconstitution sequence of the first vector consists in a splicing donor, the reconstitution sequence of the first vector consists in a splicing donor; the second vector comprises a first reconstitution sequence at the 5' end of CDS2 and a second reconstitution sequence at the 3' end of CDS2, the first reconstitution sequence being a splicing acceptor and the second being a splicing donor; the reconstitution sequence of the third vector consists in a splicing acceptor. (B3) each reconstitution sequence comprises the splicing donor/acceptor arranged as in B2 and further comprises a recombinogenic region. A degradation signal is comprised in at least one of the vectors. The figure shows for each vector all the potential positions of the one or more degradation signals of the vector system, according to preferred non-limiting embodiments of the invention.

CDS, coding sequence; SD, splicing donor signal; RR: recombinogenic regions; Deg Sig; degradation signals (see Table 2); SA, splicing acceptor signal.

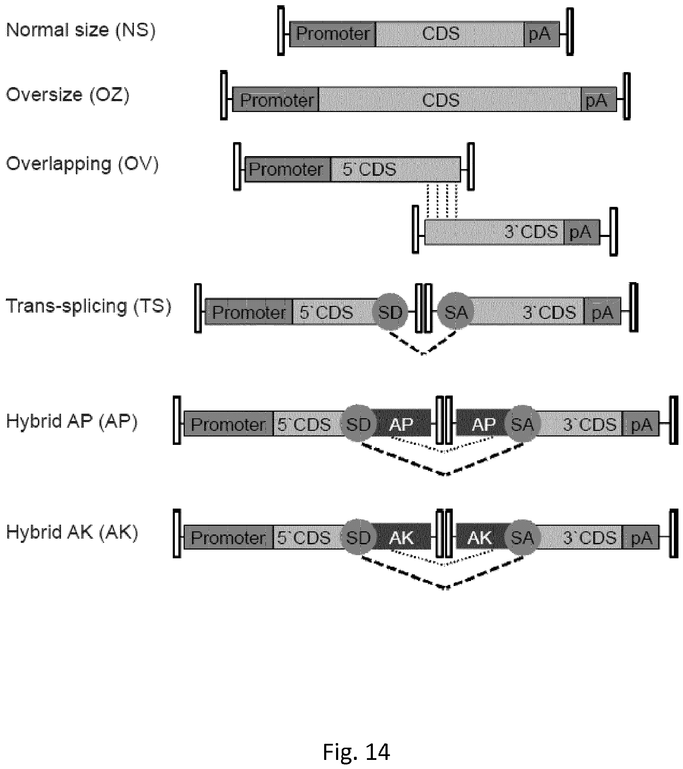

*Figure 14**. Schematic representation of prior art multiple vector-based strategies for large gene transduction.* CDS: coding sequence; pA: poly-adenilation signal; SD: splicing donor signal; SA: splicing acceptor signal; AP: alkaline phosphatase recombinogenic region; AK: F1 phage recombinogenic region. Dotted lines show the splicing occurring between SD and SA, pointed lines show overlapping regions available for homologous recombination. Normal size and oversize AAV vector plasmids contained full length expression cassettes including the promoter, the full-length transgene CDS and the poly-adenilation signal (pA). The two separate AAV vector plasmids (5' and 3') required to generate dual AAV vectors contained either the promoter followed by the N-terminal portion of the transgene CDS (5' plasmid) or the C-terminal portion of the transgene CDS followed by the pA signal (3' plasmid).

### DETAILED DESCRIPTION OF THE INVENTION

### MATERIALS AND METHODS

### Generation of plasmids

The plasmids used for AAV vector production were all derived from the dual hybrid AK vector plasmids encoding either the human *ABCA4,* the human *MYO7A* or the EGFP reporter protein containing the inverted terminal repeats (ITR) of AAV serotype 2 ¹⁴.

The AK recombinogenic sequence ¹⁴ contained in the vector plasmids encoding *ABCA4* was replaced with three different recombinogenic sequences derived from the alkaline phosphatase gene: AP (NM_001632, bp 823-1100, ¹⁴); AP1 (XM_005246439.2, bp1802-1516 ²⁰); AP2 (XM_005246439.2, bp 1225-938 ²⁰).

Dual AAV vector plasmids bearing heterologous ITR from AAV serotype 2 (ITR2) and ITR from AAV serotype 5 (ITR5) in the 5:2-2:5 configuration were generated by replacing the left ITR2 in the 5'-half vector plasmid and the right ITR2 in the 3'-half vector plasmids, respectively, with ITR5 (NC_006152.1, bp 1-175). Dual AAV vector plasmids bearing heterologous ITR2 and ITR5 in the 2:5 or 5:2 configurations were generated by replacing either the right or the left ITR2 with the ITR5, respectively. The pAAV5/2 packaging plasmid containing Rep5 (NC_006152.1, bp 171-2206) and the AAV2 Cap (AF043303 bp2203-2208) genes (Rep5Cap2), was obtained from the pAAV2/2 packaging plasmid, containing the Rep (AF043303 bp321-1993) and Cap (AF043303 bp2203-2208) genes from AAV2 (Rep2Cap2), by replacing the Rep2 gene with the Rep5 open reading frame from AAV5 (NC_006152.1, bp 171-2206).

The pZac5:5-CMV-EGFP plasmid containing the EGFP expression cassette with the ITR5 was generated from the pAAV2.1-CMV-EGFP plasmid, containing the ITR2 flanking the EGFP expression cassette ⁴⁵.

Degradation signals were cloned in dual AAV hybrid AK vectors encoding for *ABCA4* as follows: in the 5'-half vector plasmids between the AK sequence and the right ITR2; in the 3'-half vector plasmids between the AK sequence and the splice acceptor signal. Details on degradation signal sequences can be found in Table 2.

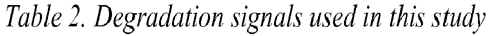

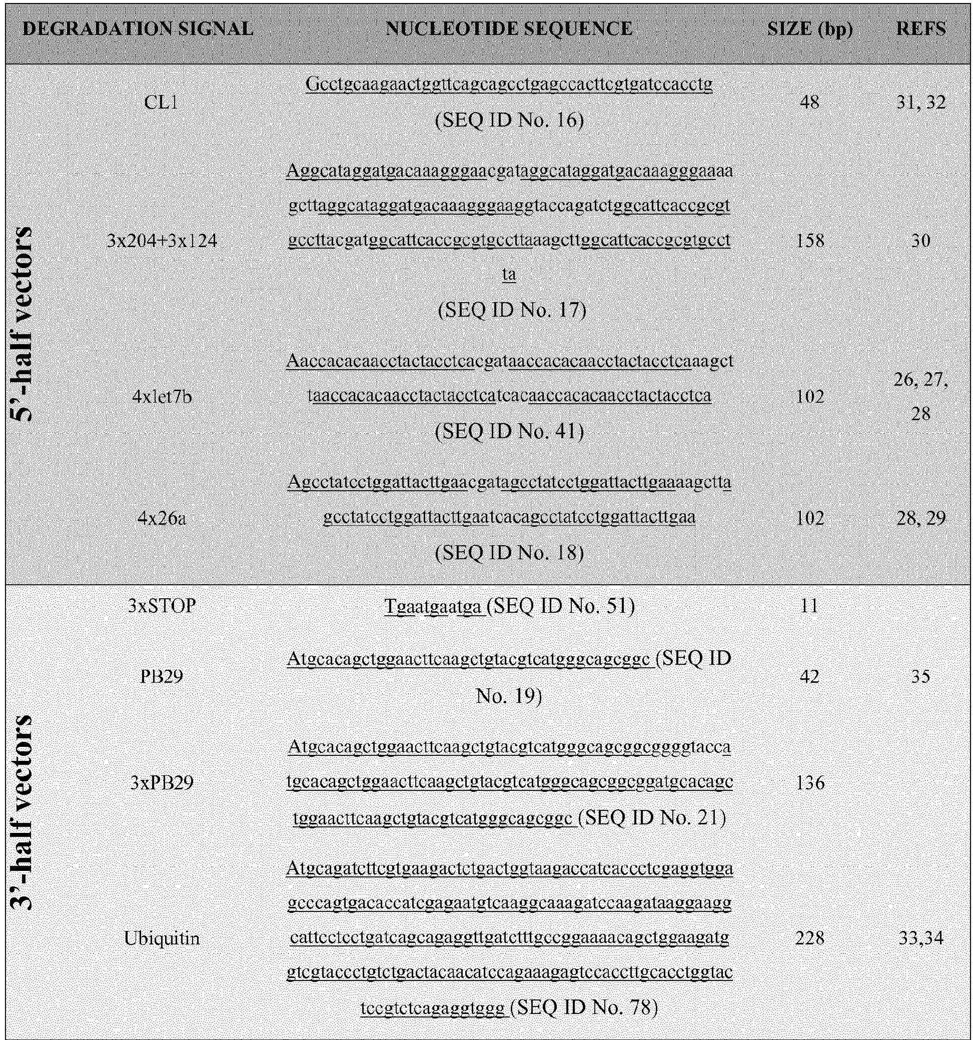

The sequences underlined correspond to the degradation signals; for degradation signals including repeated sequences, not underlined nucleotides are shown which have been included inbetween repeated sequences for cloning purposes.

The ABCA4 protein expressed from dual AAV vectors is tagged with 3xflag at both N- (amino acidic position 590) and C-termini for the experiments shown in Fig. 3 and 4 and Fig. 6, and at the C-terminus alone for the experiments in Fig. 2 and 8a.

Dual AAV hybrid vectors sets encoding for *ABCA4* used in this study included either the ubiquitous CMV ⁴⁶ or the PR-specific human G protein-coupled receptor kinase 1 (GRK1) ⁴⁷ promoters, while dual AAV hybrid vectors encoding for *MYO7A* included the ubiquitous CBA promoter ³⁹.

### AAV vector production and characterization

The AAV vector large preparations were produced by the TIGEM AAV Vector Core by triple transfection of HEK293 cells followed by two rounds of CsCl2 purification. AAV vectors bearing homologous ITR2 were obtained as previously described ⁴⁸.

To obtain AAV vectors bearing heterologous ITR2 and ITR5 a suspension of 1.1×10⁹ low-passage HEK293 cells was quadruple-transfected by calcium phosphate with 500 µg of pDeltaF6 helper plasmid which contains the Ad helper genes ⁴⁹, 260 µg of pAAV cis-plasmid and different amounts of Rep2Cap2 and Rep5 packaging constructs. The amount of Rep2Cap2 and Rep5 packaging constructs was as follows:
(i) PROTOCOL A: 130 µg of each Rep5 and Rep2Cap2 (ratio 1:1)
(ii) PROTOCOL B: 90 µg of Rep5 and 260 µg of Rep2Cap2 (ratio 1:3)
(iii) PROTOCOL C: 26 µg of Rep5 and 260 µg of Rep2Cap2 (ratio 1:10)

Each AAV preparation was then purified according to the published protocol ⁴⁸.

The protocols described below were used for the Rep competition experiments:
1- to assess Rep5 competition with Rep2 for production of AAV vectors with ITR2, HEK293 cells were either quadruple-transfected by calcium phosphate with pDeltaF6, pAAV2.1-CMV-EGFP cis, the Rep2Cap2 and Rep5Cap2 constructs at a weight ratio of 2:1:1.5:1.5 or, as a control, quadruple-transfected with the pDeltaF6, pAAV2.1-CMV-EGFP, the Rep2Cap2 packaging construct and a control irrelevant plasmid at a weight ratio of 2:1:1.5:1.5;
2- to assess Rep2 competition with Rep5 for production of AAV vectors with ITR5, HEK293 cells were either quadruple-transfected by calcium phosphate with pDeltaF6, pZac5:5-CMV-EGFP, the Rep5Cap2 and Rep2Cap2 constructs at a weight ratio of 2:1:1.5:1.5 or, as a control, quadruple-transfected with pDeltaF6, pZac5:5-CMV-EGFP, the Rep5 construct and a control irrelevant plasmid at a weight ratio of 2:1:1.5:1.5.

For the large-scale AAV vector preparations physical titres [genome copies (GC)/mL] were determined by averaging the titre achieved by PCR quantification using TaqMan (Applied Biosystems, Carlsbad, CA, USA) ⁴⁸ with a probe annealing on ITR2 and that obtained by dot-blot analysis ⁵⁰ with a probe annealing within 1 kb from ITR2. For the large-scale AAV vector preparations produced with different Rep5:Rep2Cap2 weight ratio, physical titres [genome copies (GC)/mL] were determined by PCR quantification using TaqMan with a probe annealing on ITR2. For the AAV vector preparations used in the competition experiments physical titres [genome copies (GC)] were determined by PCR quantification using TaqMan with a probe annealing on the bovine growth hormone (BGH) polyadenilation signal, included in the *EGFP-*expressing cassette packaged in the AAV vectors.

### AAV infection of HEK293 cells

AAV infection of HEK293 cells was performed as previously described ¹⁴. AAV2 vectors bearing heterologous ITR2 and ITR5 and produced according to Protocol C were used to infect HEK293 cells with a multiplicity of infection (m.o.i) of 1×10⁴ GC/cell of each vector (2×10⁴ total GC/cell when the inventors used dual AAV vectors at a 1:1 ratio) calculated considering the lowest titre achieved for each viral preparation. Infections with AAV2/2 bearing recombinogenic regions and degradation signals were carried out with a m.o.i of 5×10⁴ GC/cell of each vector (1×10⁵ total GC/cell in the case of dual AAV vectors at 1:1 ratio) calculated considering the average titre between TaqMan and dot-blot.

For the experiments using 5'-half vectors containing miR target sites, cells were transfected using calcium phosphate 4 hours prior to infection with the corresponding miR mimics (50 nM; miRIDIAN microRNA mimic hsa-let-7b-5p, hsa-miR-204-5p, hsa-miR-124-3p and hsa-miR-26a-5p; Dharmacon, Lafayette, CO, USA).

### Subretinal injection of AAV vectors in mice and pigs

Mice were housed at the Institute of Genetics and Biophysics animal house (Naples, Italy), maintained under a 12-h light/dark cycle (10-50 lux exposure during the light phase). C57BL/6 mice were purchased from Harlan Italy SRL (Udine, Italy). Pigmented *Abca4-*/*-* mice were generated through successive crosses of albino *Abca4-*/*-* mice¹⁴ with Sv129 mice and maintained inbred; breeding was performed crossing heterozygous mice with homozygous mice. Albino *Abca4-*/*-* mice were generated through successive crosses and backcrossed with BALB/c mice (homozygous for Rpe65 Leu450) and maintained inbred; breeding was performed crossing heterozygous mice with homozygous mice. C57BL/6 (5 week-old), pigmented *Abca4-*/*-* (5.5 month-old) and albino *Abca4-*/*-* (2.5-3-month old) mice were anesthetized as previously described⁶¹, then 1 µl of either PBS or AAV2/8 vectors was delivered subretinally to the temporal side of the retina via a trans-scleral trans-choroidal approach as described by Liang et al⁶². AAV2/5-VMD2-human Tyrosinase⁶³ (dose: 2×10⁸ GC/eye) was added to the AAV2/8 vector solution that was subretinally delivered to albino *Abca4-*/*-* mice (Fig. 8d). This allowed us to mark the RPE within the transduced part of the eyecup, which was subsequently dissected and analyzed.

The Large White Female pigs used in this study were registered as purebred in the LW Herd Book of the Italian National Pig Breeders' Association. Pigs were housed at the Cardarelli hospital animal house (Naples, Italy) and maintained under 12-hour light/dark cycle (10-50 lux exposure during the light phase). This study was carried out in accordance with the Association for Research in Vision and Ophthalmology Statement for the Use of Animals in Ophthalmic and Vision Research and with the Italian Ministry of Health regulation for animal procedures. All procedures were submitted to the Italian Ministry of Health; Department of Public Health, Animal Health, Nutrition and Food Safety. Surgery was performed under anesthesia and all efforts were made to minimize suffering. Animals were sacrificed as previously described³⁹. Subretinal delivery of AAV vectors to 3 month-old pigs was performed as previously described³⁹. All eyes were treated with 100 µl of either PBS or AAV2/8 vector solution. The AAV2/8 dose was 1×10¹¹ GC of each vector/eye therefore co-injection of dual AAV vectors at a 1:1 ratio resulted in a total dose of 2×10¹¹ GC/eye.

For the animal studies included in Figure 2c, 5b, 8, 9, 10, 11 and 12, right and left eyes were assigned randomly to the various experimental groups and the researchers conducting and quantifying the experiments were blind to the treatment received by the animals.

### Western blot analysis

For Western blot analysis HEK293 cells, mouse and pig retinas were lysed in RIPA buffer (50 mM Tris-HCl pH 8.0, 150 mM NaCl, 1% NP40, 0.5% Na-Deoxycholate, 1 mM EDTA pH 8.0, 0.1% SDS). Lysis buffers were supplemented with protease inhibitors (Complete Protease inhibitor cocktail tablets; Roche) and 1 mM phenylmethylsulfonyl. After lysis, samples of cells containing *MYO7A* were denatured at 99°C for 5 min in IX Laemli sample buffer; samples containing ABCA4 were denatured at 37°C for 15 min in IX Laemli sample buffer supplemented with 4 M urea. Lysates were separated by 6-7% (ABCA4 and *MYO7A* samples, respectively) or 8% (WB in Fig. 5b) SDS-polyacrylamide gel electrophoresis, The antibodies used for immuno-blotting are as follows: anti-3xflag (1:1000, A8592; Sigma-Aldrich); anti-*MYO7A* (1:500, polyclonal; Primm Srl, Milan, Italy) generated using a peptide corresponding to aminoacids 941-1070 of the human *MYO7A* protein; anti-Filamin A (1:1000, catalog #4762; Cell Signaling Technology, Danvers, MA, USA); anti-Dysferlin (1:500, Dysferlin, clone Ham1/ 7B6, MONX10795; Tebu-bio, Le Perray-en-Yveline, France). The quantification of ABCA4 and *MYO7A* bands detected by Western blot was performed using ImageJ software (free download available at http://rsbweb.nih.gov/ij/). For the in vitro experiments performed with AAV bearing heterologous ITR2 and ITR5, the intensity of the full-length ABCA4 and *MYO7A* bands was normalized to either that of the truncated protein product in the corresponding lane or to that of Filamin A bands, while the intensity of the shorter ABCA4 and *MYO7A* proteins bands was normalized to that of Filamin A bands. The intensity of ABCA4 bands achieved with AAV vectors bearing degradation signals or homology regions was normalized to that of Filamin A bands for the *in vitro* experiments or Dysferlin bands for the *in vivo* experiments. Quantification of the Western blot experiments has been performed as follows:
- Fig. 2a-b: the intensity of the ABCA4 band was normalized to that of Filamin A band in the corresponding lane. Normalized ABCA4 expression was then expressed as percentage relative to dual AAV hybrid AK vectors;
- Fig. 2c: the intensity of the ABCA4 band (a.u.) was calculated as fold of increase relative to the mean intensity measured at the same level in the negative control lanes of each gel (the measurement of the negative control sample in lane 7 of the lower left panel was excluded from the analysis given the exceptionally high background signal). Values for each group are represented as mean ± standard error of the mean (s.e.m.);
- Fig. 3b-d: the full-length ABCA4 and truncated protein band intensities were divided by those of the Filamin A bands or the intensity of the full-length ABCA4 protein bands was divided by that of the truncated protein bands in the corresponding lane. Values are represented as: mean ± s.e.m.;
- Table 5: full-length ABCA4 and truncated protein band intensities were measured in cells co-infected with 5'- and 3'-half vectors. The ratio between the intensity of full-length ABCA4 and truncated protein bands in the presence of either the corresponding mimic or a scramble mimic was calculated. Values represent mean ± s.e.m. of the ratios from three independent experiments;
- Table 6: full-length ABCA4 and truncated protein band intensities were measured in cells co-infected with 5'- and 3'-half vectors. The ratio between the intensity of the full-length ABCA4 and truncated bands from vectors either with or without the degradation signals was calculated. Values represent mean ± s.e.m. of the ratios from three independent experiments.
- Fig. 8a: the intensity of the ABCA4 band (a.u.) was calculated as fold of increase relative to the mean background intensity measured in the negative control lanes of the corresponding gel. Values are expressed as mean ± s.e.m.

### Southern blot analysis

Three ×10¹⁰ GC of viral DNA were extracted from AAV particles. To digest unpackaged genomes, the vector solution was resuspended in 240 µl of PBS pH 7.4 19 (GIBCO; Invitrogen S.R.L., Milan, Italy) and then incubated with 1 U/µl of DNase I (Roche) in a total volume of 300 µl containing 40 mM TRIS-HCl, 10 mM NaCl, 6 mM MgCl2, 1 mM CaCl2 pH 7.9 for 2 h at 37°C. The DNase I was then inactivated with 50 mM EDTA, followed by incubation with proteinase K and 2.5% N-lauroyl-sarcosil solution at 50°C for 45 min to lyse the capsids. The DNA was extracted twice with phenol-chloroform and precipitated with two volumes of ethanol 100 and 10% sodium acetate (3 M, pH 7). Alkaline agarose gel electrophoresis and blotting were performed as previously described (Sambrook & Russell, 2001 Molecular Cloning). Ten microlitres of the 1 kb DNA ladder (N3232L; New England Biolabs, Ipswich, MA, USA) were loaded as molecular weight marker. Two different double strand DNA fragments were labelled with digoxigenin-dUTP using the DIG high prime DNA labelling and detection starter kit (Roche) and used as probes. The 5' probe (768 bp) was generated by double digestion of the pZac2.1-CMV-ABCA4_5' plasmid with Spel and NotI; the 3' probe (974 bp) was generated by double digestion of the pZac2.1-ABCA4_3'_3xflag_SV40 plasmid with ClaI and MfeI. Prehybridization and hybridization were performed at 65°C in Church buffer (Sambrook & Russel, 2001 Molecular cloning) for 1 h and overnight, respectively. Then, the membrane (Whatman Nytran N, charged nylon membrane; Sigma-Aldrich, Milan, Italy) was first washed for 30 min in SSC 29-0.1% SDS, then for 30 min in SSC 0.59-0.1% SDS at 65°C, and then for 30 min in SSC 0.19-0.1% SDS at 37°C. The membrane was then analyzed by chemiluminescence detection by enzyme immunoassay using the DIG DNA Labeling and Detection Kit (Roche).

### Histological analysis

Mice were euthanized, and their eyeballs were then harvested and fixed overnight by immersion in 4% paraformaldehyde (PFA). Before harvesting the eyeballs, the temporal aspect of the sclerae was marked by cauterization, in order to orient the eyes with respect to the injection site at the moment of the inclusion. The eyeballs were cut so that the lens and vitreous could be removed while leaving the eyecup intact. Mice eyecups were infiltrated with 30% sucrose for cryopreservation and embedded in tissue-freezing medium (O.C.T. matrix; Kaltek, Padua, Italy). For each eye, 150-200 serial sections (10 µm thick) were cut along the horizontal plane and the sections were progressively distributed on 10 slides so that each slide contained 15 to 20 sections, each representative of the entire eye at different levels. The sections were stained with 4',6'-diamidino-2-phenylindole (Vectashield; Vector Lab, Peterborough, United Kingdom) and were monitored with a Zeiss Axiocam (Carl Zeiss, Oberkochen, Germany) at different magnifications.

Pigs were sacrificed, and their eyeballs were harvested and fixed overnight by immersion in 4% PFA. The eyeballs were cut so that the lens and vitreous could be removed, leaving the eyecups in place. The eyecups were gradually dehydrated by progressively infiltrating them with 10%, 20%, and 30% sucrose. Tissue-freezing medium (O.C.T. matrix; Kaltek) embedding was performed. Before embedding, the swine eyecups were analyzed with a fluorescence stereomicroscope (Leica Microsystems GmbH, Wetzlar, Germany) in order to localize the transduced region whenever an EGFP-encoding vector was administered. For each eye, 200-300 serial sections (12 µm thick) were cut along the horizontal meridian and the sections were progressively distributed on glass slides so that each slide contained 6-10 sections. Section staining and image acquisition were performed as described for mice.

### Cone immunofluorescence staining

Frozen retinal sections were washed once with PBS and then permeabilized for 1 hr in PBS containing 0.1% Triton X-100. Blocking solution containing 10% normal goat serum (Sigma-Aldrich) was applied for 1 hr. Primary antibody [anti-human CAR^{66,67}, which also recognises the porcine CAR ("Luminaire founders"-hCAR, 1:10,000; kindly provided by Dr. Cheryl M. Craft, Doheny Eye Institute, Los Angeles, CA)] was diluted in PBS and incubated overnight at 4°C. The secondary antibody (Alexa Fluor 594, anti-rabbit, 1:1,000; Molecular Probes, Invitrogen, Carlsbad, CA) was incubated for 45 min. Sections stained with the anti-CAR antibodies were analyzed at 63x magnification using a Leica Laser Confocal Microscope System (Leica Microsystems GmbH), as previously described⁶⁴. Briefly, for each eye six different z-stacks from six different transduced regions were taken. For each z-stack, images from single plans were used to count CAR+ /EGFP ± cells. In doing this, the inventors carefully moved along the Z-axis to distinguish one cell from another and thus to avoid to count twice the same cell. For each retina the inventors counted the CAR-positive (CAR+)/EGFP-positive (EGFP+) cells on total CAR+ cells. The inventors then calculated the average number of CAR+ /EGFP + cells of the three eyes of each experimental group.

### EGFP quantification

Fluorescence intensity in PR was rigorously and reproducibly quantified in an unbiased manner as previously described⁶⁴. Individual color channel images were taken using a Leica microscope (Leica Microsystems GmbH). TIFF images were gray-scaled with image analysis software (LAS AF lite; Leica Microsystems GmbH). Six images of each eye were analyzed at 20x magnification by a masked observer. PR (outer nuclear layer + OS) were selectively outlined in every image, and the total fluorescence for the enclosed area was calculated in an unbiased manner using the image analysis software. The fluorescence in PR was then averaged from six images collected from separate retinal sections from each eye. The inventors then calculated the average fluorescence of the three eyes of each experimental group.

### Quantification of lipofuscin autofluorescence

For lipofuscin fluorescence analysis, eyes were harvested from pigmented *Abca4+*/*-* and *Abca4-*/- mice at 3 months after AAV injection. Mice were dark-adapted over-night and sacrificed under dim red-light. For each eye, four overlapping pictures from the temporal side of three sections from different regions of the eye were taken using a Leica DM5000B microscope equipped with a TX2 filter (excitation: 560±40 nm; emission: 645±75) ⁷¹⁻⁷⁵ and under a 20X objective. The four images for each section were then combined in a single montage used for further fluorescence analysis. Intensity of lipofuscin fluorescence (red signal) in each section was automatically calculated using the ImageJ software and was then normalized for the length of the RPE underlying the area of fluorescence.

### Transmission electron microscopy

For electron microscopy analyses eyes were harvested from albino *Abca4-*/*-* and Abca4+/+ mice at 3 months after AAV injection. Eyes were fixed in 0.2% glutaraldehyde-2% paraformaldehyde in 0.1 M PHEM buffer pH 6.9 (240 mM PIPES, 100 mM HEPES, 8 mM MgCl2, 40 mM EGTA) overnight and then rinsed in 0.1 M PHEM buffer. Eyes were then dissected under light microscope to select the tyrosinase-positive portions of the eyecups. The transduced portion of the eyecups were subsequently embedded in 12% gelatin, infused with 2.3 M sucrose and frozen in liquid nitrogen. Cryosections (50 nm) were cut using a Leica Ultramicrotome EM FC7 (Leica Microsystems) and extreme care was taken to align PR connecting cilia longitudinally. To avoid bias in the attribution of morphological data to the various experimental groups, counts of lipofuscin granules were performed by a masked operator (Dr. Roman Polishchuk) using the iTEM software (Olympus SYS, Hamburg, Germany). The 'Touch count' module of the iTEM software was used to count the number of lipofuscin granules in 25 µm² areas (at least 40) distributed randomly across the RPE layer. The granule density was expressed as number of granules per 25 µm².

### Electroretinogram Recordings

Electrophysiological recordings in mice and pigs were performed as detailed in (68) and in (69), respectively.

### Statistical analysis

p-values ≤ 0.05 were considered statistically significant. One-way ANOVA (R statistical software) with post-hoc Multiple Comparison Procedure was used to compare data depicted in Figure 2b (pANOVA=1,2 × 10⁻⁶), 2c (pANOVA= 0,326), 8c (pANOVA=1,5 × 10⁻¹⁰), 8d (pANOVA= 0,034) and 9a (pANOVA a-wave: 0,5; pANOVA b-wave: 0,8) and Table 6 (pANOVA= 0,0135). As the counts of lipofuscin granules (Fig. 8d) are expressed as discrete numbers, these were analyzed by deviance from a Negative Binomial generalized linear models⁶⁵. The statistically significant differences between groups determined with the post-hoc Multiple Comparison Procedure are the following: Fig. 2b: AP vs AK: 1,08 × 10⁻⁵; AP1 vs AK: 0,05; AP2 vs AK: 0,17; AP1 vs AP: 1,8 × 10⁻⁶; AP2 vs AP: 2,8 × 10⁻⁶; AP2 vs API: 0,82. Fig. 8c: *Abca4+*/*-* not inj vs *Abca4-*/*-* not inj: 0,00; *Abca4-*/*-* not inj vs *Abca4-*/*-* AAV5'+3': 9,3 × 10⁻⁵; *Abca4+*/*-* not inj vs *Abca4-*/*-* AAV5'+3': 4 × 10⁻⁶. Fig. 8d: *Abca4-*/*-* PBS vs *Abca4-*/*-* AAV5'+3': 0,01; Abca4+/+ PBS vs *Abca4-*/*-* AAV5'+3': 0,37; Abca4+/+ not inj vs *Abca4-*/*-* AAV5'+3': 0,53; Abca4+/+ PBS vs *Abca4-*/*-* PBS: 0,05; Abca4+/+ not inj vs *Abca4-*/*-* PBS: 0,03; Abca4+/+ not inj vs Abca4+/+ PBS: 0,76. Table 6: 3xSTOP vs no degradation signal: 0,97; 3xSTOP vs PB29: 1,0; 3xSTOP vs 3xPB29: 0,15; 3xSTOP vs ubiquitin: 0,10; PB29 vs no degradation signal: 1,0; PB29 vs 3xPB29: 0,1; PB29 vs ubiquitin: 0,07; 3xPB29 vs no degradation signal: 0,06; 3xPB29 vs ubiquitin: 1,0; ubiquitin vs no degradation signal: 0,04.

The Student's t-test was used to compare data depicted in Figure 3c, d and f.

### RESULTS

### Dual AAV hybrid vectors which include the AP1, AP2 or AK recombinogenic regions show efficient transduction

The inventors evaluated several multiple vector strategies as depicted in Fig. 1 and 13.

In particular, they evaluated in parallel the transduction efficacy of dual AAV hybrid vectors with different regions of homology. For this purpose the inventors generated dual AAV2/2 hybrid vectors that include the *ABCA4*-3xflag coding sequence, under the control of the ubiquitous CMV promoter, and either the AK ¹⁴, AP ¹⁴, AP1 or AP2 ²⁰ regions of homology (Fig. 7). The inventors used these vectors to infect HEK293 cells [multiplicity of infection, m.o.i.: 5×10⁴ genome copies (GC)/cell of each vector]. Cell lysates were analysed by Western blot with anti-3xflag antibodies to detect ABCA4-3xflag (Fig. 2). Each of the dual AAV hybrid vectors sets resulted in expression of full-length proteins of the expected size that were not detected in the lanes loaded with negative controls (Fig. 2a). Quantification of ABCA4 expression (Fig. 2b) showed that infection with dual AAV hybrid AP1 and AP2 vectors resulted in slightly higher levels of transgene expression than with dual AAV hybrid AK vectors and all significantly outperformed dual AAV hybrid AP vectors ¹⁴. The inventors have previously found that the efficiency of dual AAV vectors which rely on homologous recombination is lower in terminally-differentiated cells as PR than in cell culture ¹⁴. The inventors therefore evaluated PR-specific transduction levels in C57BL/6 mice following subretinal administration of dual AAV AK, AP1 and AP2 vectors which include the PR-specific human G protein-coupled receptor kinase 1 (GRK1) promoter (dose of each vector/eye: 1.9×10⁹ GC; Fig. 2c). One month after vector administration the inventors detected ABCA4 protein expression more consistently in retinas treated with dual AAV hybrid AK than AP1 or AP2 vectors (Fig. 2c).

### Inclusion of heterologous ITR in AAV vectors affects their production yields and does not reduce levels of truncated protein products

To test if the use of heterologous ITR improve the productive directional concatemerization of dual AAV vectors, the inventors generated dual AAV2/2 hybrid AK vectors that included either *ABCA4*-3xflag or *MYO7A*-HA coding sequences with heterologous ITR2 and ITR5 in either the 5:2 (left ITR from AAV5 and right ITR from AAV2) or the 2:5 (left ITR from AAV2 and right ITR from AAV5) configuration (Fig. 1). The production of dual AAV vectors bearing heterologous ITR2 and ITR5 requires the simultaneous expression of the Rep proteins from AAV serotypes 2 and 5 which cannot cross-complement virus replication ²³. Indeed, it has been shown that Rep2 and Rep5 can bind interchangeably to ITR2 or ITR5, although less efficiently than to homologous ITR, however they cannot cleave the terminal resolution sites of the ITR from the other serotype ³⁶. Therefore, before generating dual AAV hybrid AK vectors with heterologous ITR2 and ITR5, the inventors assessed the potential competition of (i) Rep5 with Rep2 in the production of AAV2/2-CMV-EGFP vectors (i.e. vectors with homologous ITR2) and (ii) Rep2 with Rep5 in the production of AAV5/2-CMV-EGFP vectors (i.e. vectors with homologous ITR5), using the same amount of the Rep5Cap2 and Rep2Cap2 packaging constructs (ratio 1:1). Indeed, when the Rep5Cap2 packaging construct is provided in addition to Rep2Cap2, the total yields of AAV2/2-CMV-EGFP vectors are reduced to 42% of those of control preparations obtained when only Rep2Cap2 is provided as packaging construct (average of 4 independent preps of each type, p Student's t-test <0.05). Conversely, no significant differences were found in the total yields of AAV5/2-CMV-EGFP preps obtained when Rep2Cap2 was added to Rep5Cap2, which were 83% of those obtained when Rep5Cap2 was the only packaging construct transfected (average of 4 independent preps of each type, no significant differences were found using Student's t-test). Given the competition of Rep5 with Rep2 in the production of vectors with ITR2, the inventors tested three different ratios between Rep5 and the Rep2Cap2 packaging constructs in the production of AAV with heterologous ITR2 and ITR5 (Protocol A with 1:1, Protocol B with 1:3 and Protocol C with 1:10 Rep5/Rep2Cap2 ratio). As shown in Table 3, viral titres determined by PCR quantification using a probe annealing to ITR2 progressively increased when the amount of Rep5 was decreased, with the best titre obtained with Protocol C.

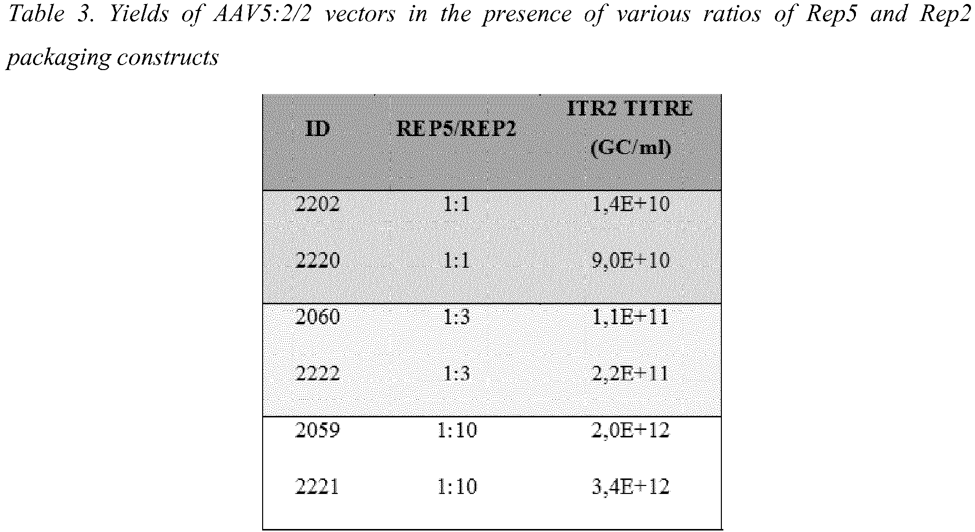

ID: identification number of AAV5:2/2 vectors; GC: genome copies.

These results confirmed the competition of Rep5 with Rep2 during the production of vectors with ITR2 and led us to follow Protocol C for the production of AAV vectors with heterologous ITR2 and ITR5. However, several AAV preparations obtained with this strategy revealed: (i) up to 6-fold lower titres determined on ITR2 than titres determined on a transgenic sequence in between the ITR (Table 4) which could suggest that the integrity of ITR2 is compromised and (ii) a mean reduction of about 6-fold in the total yields of AAV vectors with heterologous ITR2 and ITR5 compared to those containing homologous ITR2 (Table 4).

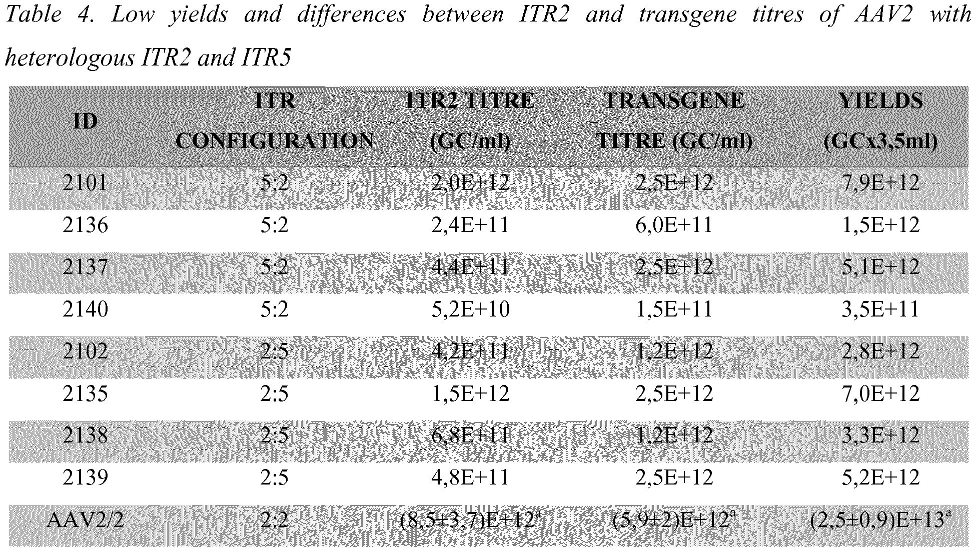

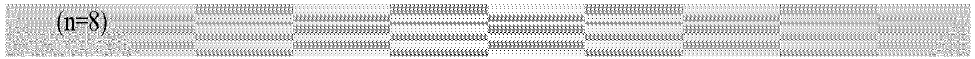

ID: identification number of AAV vectors; GC: genome copies.^{a} Values represent mean±SEM. However, Southern blot analysis of AAV preparation with heterologous ITR revealed no evident alteration of genome integrity (Fig. 3a).

To test if the inclusion of heterologous ITR in dual AAV hybrid AK vectors enhanced the formation of tail-to-head productive concatemers and full-length protein transduction while reducing the production of truncated proteins, the inventors infected HEK293 cells with dual AAV hybrid vectors encoding for either *ABCA4* or *MYO7A* with either heterologous ITR2 and ITR5 (in the 5:2/2:5 configuration) or homologous ITR2 (Fig. 3b, 3e).

Given the difference between the ITR2 and transgene titres for vectors with heterologous but not homologous ITR (Table 4), the inventors infected cells with 10⁴ genome copies (GC)/cell of each vector based on either ITR2 or transgene titres. Western blot analysis of HEK293 cells infected with dual AAV vectors based on ITR2 titers, using anti-3xflag (to detect ABCA4-3xflag, Fig. 3b) or anti-Myo7a (Fig. 3e) antibodies, showed that the inclusion of heterologous ITR2 and ITR5 resulted in higher levels of both full-length and truncated protein than homologous ITR2 (Fig. 3b, c, d, f). However this was not observed when HEK293 cells were infected with the same dual AAV vector preps based on the transgene titre (Fig. 3b, d). In conclusion, the ratio between full-length and truncated protein expression was similar regardless of the ITR included in the vectors (Fig. 3 c, d, f) and of the vector titre used to dose cells (Fig. 3b, c, d).

### CL1 degron in the 5'-half vector decreases the production of truncated protein products

To selectively reduce the levels of truncated protein products produced by each 5'- and 3'-half of dual AAV hybrid vectors ¹⁴, the inventors placed putative degradation sequences in the 5'-half vector after the splicing donor signal between AK and the right ITR, and in the 3'-half vector between AK and the splicing acceptor signal (Fig. 1). Thus, the degradation signal will be included in the truncated but not in the full-length protein which results from a spliced mRNA. As degradation signals in the 5'-half vectors the inventors have included: (i) the CL1 degron (CL1), (ii) 4 copies of the miR-let7b target site (4xLet7b), (iii) 4 copies of the miR-26a target site (4x26a) or (iv) the combination of 3 copies each of miR-204 and miR-124 target sites (3x204+3x124) (Table 2). As degradation signals in the 3'-half vectors the inventors have included: (i) 3 stop codons (STOP), (ii) PB29 either in a single (PB29) or in three tandem copies (3xPB29) or (iii) ubiquitin (Table 2). The inventors generated dual AAV2/2 hybrid AK vectors encoding for ABCA4 including the various degradation signals and evaluated their efficacy after infection of HEK293 cells [m.o.i.: 5 × 10⁴ genome copies (GC)/cell of each vector]. Since miR-let7b, miR-26a, miR-204 and miR-124 are poorly expressed or completely absent in HEK293 cells (Ambion miRNA Research Guide and ³⁷), to test the silencing of the construct containing target sites for these miR, the inventors transfected cells with miR mimics (i.e. small, chemically modified double-stranded RNAs that mimic endogenous miR ³⁸) prior to infection with the AAV2/2 vectors containing the corresponding target sites. To define the concentration of miR mimics required to achieve silencing of a gene containing the corresponding miR target sites, the inventors used a plasmid encoding for the reporter EGFP protein and containing the miR target sites before the polyadenylation signal (data not shown). The same experimental settings were used for further evaluation of the miR target sites in the context of dual AAV hybrid AK vectors. The inventors found that inclusion of miR-204+124 and 26a target sequences in the 5'-half of dual AAV hybrid AK vectors reduced albeit did not abolish the expression of the truncated protein products without affecting full-length protein expression (Fig. 4). Differently, the inclusion of miR-let7b target sites was not effective in reducing truncated protein expression (Fig. 4).

Notably, as shown in figure 5a, the inventors found that the inclusion of the CL1 degradation signal in the 5'-half vector reduced truncated protein expression to undetectable levels without affecting full-length protein expression (Fig. 5a). Since differences in the tissue-specific expression of enzymes of the ubiquitination pathway that mediate CL1 degradation ³¹ may account for changes in CL1 efficacy, the inventors further evaluated the efficacy of the CL1 degron in the pig retina, which has a size and structure similar to human ^{19, 30, 39, 40} and is therefore an excellent pre-clinical large animal model to evaluate vector safety and efficiency. To this aim, the inventors injected subretinally in Large White pigs AAV2/8 dual AAV hybrid AK vectors (of which the 5'-half vector included or not the CL1 sequence) encoding for *ABCA4* (dose of each vector/eye: 1 × 10¹¹ GC). Notably, the inventors found that the inclusion of the CL1 degradation signal in the 5'-half vector resulted in a significant reduction of truncated protein expression below the detection limit of the Western blot analysis without affecting full-length protein expression (Fig. 5b). Among the degradation signals tested in the 3'-half vector the inventors found that STOP codons did not affect truncated protein production. Differently, PB29 (either in a single or in three tandem copies) and Ubiquitin were all effective in reducing truncated protein expression. However, while Ubiquitin abolished also full-length protein expression, PB29 affected full-length protein production to a lesser extent (Fig. 6).

Among the degradation signals tested in the 3'-half vector the inventors identified three (PB29, 3xPB29 and ubiquitin) that reduced both the levels of truncated protein products and of full-length proteins (Fig. 6 and Tables 5 and 6).

**Table 5. Quantification of full-length ABCA4 relative to truncated protein expression from Western blot analysis of HEK293 cells infected with dual AA V hybrid vectors including miR target sites in the 5'-half vector.**

| **miR TARGET SITES** | **FULL-LENGTH ABCA4 /** | **TRUNCATED PROTEIN** |
|---|---|---|
| | **+SCRAMBLE** | **+miR** |
| 5'-miR-let7b +3' | 1.2 ± 0,3 | 0.8±0.3 |
| 5'-miR-204+124 +3' | 1.8 ± 0.5 | 2.7 ± 0.9 |
| 5'-miR-26a +3' | 1,9 ± 0.8 | 2.5 ± 1,1 |

| | | |
|---|---|---|
| Values represent mean ± s.e.m. of the ratios (from three independent experiments) between the intensity of full-length ABCA4 and truncated protein bands in the presence of either the corresponding mimic or a scramble mimic. Ratios in the presence of either the scramble or the corresponding mimic for each pair of vectors were compared using Student's ttest and no significant differences were found. | | |

**Table 6: Quantification of full-length ABCA4 and truncated protein expression from Western blot analysis of HEK293 cells infected with dual AAV hybrid vectors including degradation signals in the 3 '-halfvector.**

| **PROTEIN DEGRADATION SIGNALS** | **FULL LENGHTC ABCA4 / TRUNCATED** | |
|---|---|---|
| | **5'+3' NO DEGRADATION** | **5'+3' DEGRADATIO** N **SIGNAL** |
| 3xSTOP | 5,9± 1,8 | 4,9 ± 1,1 |
| PB29 | | 5,9± 1,1 |
| 3xPB29 | | 1 ± 0,3 |
| ubiquitin | | 0,6 ± 0,2 |

| | | |
|---|---|---|
| Values represent mean ± s.e.m. of the ratios (from three independent experiments) between the intensity of the full-length ABCA4 and truncated protein bands from vectors either with or without the degradation signals. More details on the statistical analysis including specific statistical values can be found in the Statistical analysis paragraph of the Materials and Methods section | | |

### Subretinal administration of improved dual AAV vectors reduces lipofuscin accumulation in the Abca4-/- retina

Based on our findings improved dual AAV hybrid-*ABCA4* vectors should include homologous ITR2, the AK region of homology and the CL1. As *ABCA4* is expressed in both rod and cone photoreceptors in humans⁷⁰, the inventors identified a suitable promoter for *ABCA4* delivery by comparing the PR transduction properties of single AAV2/8 vectors encoding *EGFP* from either the human GRK1 (G protein-coupled receptor kinase 1) or IRBP (interphotoreceptor retinoid binding protein) promoters, which have been both described to drive high levels of combined rod and cone PR transduction in various species⁵³⁻⁵⁵. Taking advantage of the pig retinal architecture which include a streak-like region with a cone:rod = 1:3⁵⁶ similar to the human macula, the inventors injected subretinally 1×10¹¹ GC/eye of either AAV2/8-GRK1- or IRBP-*EGFP* vectors in 3 month-old Large White pigs. Four weeks after the injection, the inventors analysed the corresponding retinal cryosections under a fluorescence microscope. EGFP fluorescence quantification in the PR cell layer (Fig. 10a-b) showed that both promoters give comparable levels of PR transduction (predominantly rods in this region). However, when the inventors counted the number of cones labelled with an antibody raised against cone arrestin (CAR)⁵⁷ that were also EGFP positive, they found higher although not statistically significant levels of cone PR transduction with the GRK1 promoter (Material, Fig. 10c-d). Based on this, the inventors included the GRK1 promoter in our improved dual AAV hybrid *ABCA4* vectors, and investigated their ability to both express *ABCA4* and decrease the abnormal content of A2E-containing autofluorescent lipofuscin material in the RPE of *Abca4-*/*-* mice. The inventors initially injected subretinally one month-old C57/BL6 mice with improved dual AAV vectors (dose of each vector/eye: 2×10⁹ GC) and found that 12 out of 24 (50%) injected eyes had detectable albeit variable levels of full-length ABCA4 protein by Western blot [Fig. 8a; ABCA4 protein levels in the ABCA4-positive eyes: 2,8 ± 0,7 a.u. (mean ± standard error of the mean)]. This is similar to our previous finding that a different version of the dual AAV platform resulted in 50% ABCA4-expressing eyes¹⁴. The inventors then injected 5.5 month-old pigmented *Abca4-*/- mice subretinally in the temporal region of the eye with the improved dual AAV vectors (dose of each vector/eye: 1.8×10⁹ GC). Three months later the inventors harvested the eyes and measured the levels of lipofuscin fluorescence (excitation: 560±40 nm; emission: 645±75) on retinal cryosections [in either the RPE alone or in RPE +outer segments (OS)] in the temporal region of the eye (Fig. 8b-c and Fig. 11). The inventors found that lipofuscin fluorescence intensity in this region of the eye was significantly higher in untreated *Abca4-*/*-* than in both *Abca4+*/*-* and -/- mice injected with the therapeutic dual AAV hybrid *ABCA4* vectors (Fig. 8b, c and Fig. 11). Then, using transmission electron microscopy the inventors counted the number of RPE lipofuscin granules. These were increased in 5.5-6-month old albino *Abca4-*/*-* mice injected with PBS compared to age-matched *Abca4*+/+ controls (Fig. 8d), at levels similar to those the inventors have independently measured in *Abca4-*/*-* mice either uninjected or injected with a control AAV vector (data not shown). The number of lipofuscin granules in *Abca4-*/*-* RPE was normalized 3 months post subretinal injection of improved dual AAV hybrid *ABCA4* vectors (dose of each vector/eye: 1×10⁹ GC, Fig. 8d).

### Improved dual AAV vectors are safe upon subretinal administration to the mouse and pig retina

To investigate the safety of improved dual AAV2/8 hybrid *ABCA4* vectors, the inventors injected them subretinally in both wild-type C57BL/6 mice and Large White pigs (dose of each vector/eye: 3×10⁹ and 1×10¹¹ GC, respectively). One month post-injection the inventors measured retinal electrical activity by Ganzfeld electroretinogram (ERG) and found that both the a- and b-wave amplitudes were not significantly different between mouse eyes that were injected with dual AAV hybrid *ABCA4* vectors and eyes injected with either negative control AAV vectors or PBS (Fig. 9a and Material, Fig. 12a). Similarly, the b-wave amplitude in both scotopic, photopic, maximum response and flicker ERG tests was comparable in pig eyes that were injected with dual AAV hybrid *ABCA4* vectors to those of control eyes injected with PBS (Fig. 9b and Material, Fig. 12b).

### DISCUSSION

AAV restricted packaging capacity represents one of the main obstacles to the widespread application of AAV for gene therapy of IRDs. However, recently, several groups have independently reported that dual AAV vectors effectively expand AAV cargo capacity in both the mouse and pig retina ^{14, 17, 19, 41} thus extending AAV applicability to IRDs due to mutations in genes that would not fit in a single canonical AAV vector. Here the inventors set-up to overcome some limitations associated with the use of dual AAV vectors, namely their relatively low efficiency when compared to a single vector, and the production of truncated proteins which may raise safety concerns.

Strategies aiming at increasing dual AAV genome tail-to-head concatemerization should in theory increase the levels of full-length and reduce those of truncated proteins from free single half-vectors. The inventors set to improve tail-to-head dual AAV hybrid genome concatemerization by including either optimal regions of homology or heterologous ITR. In a side-by-side evaluation of previously described regions of homology, the inventors have found that the AP1 and AP2 sequences recently published by Lostal et al. ²⁰ and the AK sequence from the F1 phage ¹⁴ drive overall similar levels of protein expression *in vitro* with dual AAV hybrid AK vectors driving more consistent ABCA4 expression in the mouse retina. Independently, the availability of different regions of homology is useful to direct proper concatemerization of triple AAV vectors to further expand AAV cargo capacity ^{20, 42}. Heterologous ITR2 and ITR5 have been successfully included in dual ^{24, 25} and triple ⁴² AAV vectors. The inventors found that the yields of AAV vectors with heterologous ITR2 and ITR5 are lower than those with homologous ITR2. The inventors also detected less vector genomes with heterologous ITR when the inventors probe their ITR2 than when the inventors probe a different region of their genome. As the inventors show that Rep5 interferes with production of vectors with ITR2, this suggests anomalies at the level of ITR2 included in AAV vectors with heterologous ITR, which are produced in the presence of Rep5, but not in AAV vectors with homologous ITR2, which are produced only in the presence of Rep2 and that showed similar titres whether the inventors probe ITR2 or a different region of the genome. These results partly differ from those previously reported where dual AAV vectors with heterologous ITR2 and ITR5 had higher transduction efficiency than vectors with homologous ITRs and apparently no production issues ^{24, 25}. Besides the different packaging constructs and production protocols, in this study the inventors used dual AAV hybrid vectors which included regions of homology between the two half-vectors as opposed to the trans-splicing system used in the previous reports which simply relies on the ITR for concatemerization ^{24, 25}. As in dual AAV hybrid vectors the reconstitution of the full-length gene is mainly mediated by the region of homology included in the vectors ¹⁶ which direct concatemer formation, this may account for the smaller increase in transgene expression the inventors observed with vectors with heterologous ITR compared to the previous studies that used trans-splicing vectors ^{24, 25}. In addition, the inventors may have overestimated the efficiency of the vectors with heterologous ITR as the inventors used them based on a titre calculated on ITR2 which is 3-6-fold lower than the one calculated on the transgenic sequence for MYO7A- and ABCA4-expressing vectors, respectively. As both titres calculated on ITR2 and on the transgenic sequence are similar between the corresponding dual AAV vectors with homologous ITR2, the inventors have used them at a 3-6-fold lower volume than those with the heterologous ITR2 and ITR5. This may explain the apparently higher levels of both full-length and truncated protein products from dual AAV vector with heterologous than with homologous ITR.

In the inventors' previous studies the inventors did not observe signs of local toxicity up to 8 months after subretinal administration of dual AAV vectors ¹⁴, however, the production of truncated protein products from single half-vectors of dual AAV might raise safety concerns. The inclusion of miR target sites in the transcript of a gene has been shown to be an effective strategy to restrict transgene expression in various tissues, including the retina ³⁰. However *in vitro* the inventors achieved a partial reduction of truncated protein production only when the inventors included target sites for miR-204+124 and 26a. Indeed, features of the mRNA external to the miR target sites may affect the efficiency of the silencing ^{43, 44}. Along this line, since the truncated protein products that derive from the 5'-half is produced from a vector that is not endowed with a canonical polyadenilation signal, it may be possible that the resulting mRNA can not undergo an efficient miR-mediated silencing. Importantly, the inventors achieved complete degradation of the truncated protein product from the 5'-half vector by inclusion of the CL1 degron. The inventors showed that this signal is effective both *in vitro* and in the pig retina, indicating that the enzymes of the degradative pathway required for CL1 activity are expressed in various cell types. As the truncated protein product from the 3'-half vector is less abundant than that produced by the 5'-half vector (Fig. 6), its presence should raise less safety concerns. Data presented here in the mouse and pig retina support the safety of improved dual AAV vectors.

Notably, the inventors found that subretinal administration of improved dual AAV vectors, under the control of the GRK1 promoter, which provides high levels of combined rod and cone transduction, results in effective ABCA4 delivery in mice, although at variable levels. This could be due to both the inherent variability of the subretinal injection in the small murine eye and the overall lower efficacy of the dual AAV system compared to a single AAV vector¹⁴. Despite this variability, the inventors found that dual AAV mediated ABCA4 delivery results in significant lipofuscin reduction in the *Abca4-*/*-* retina suggesting that a wide range of transgene expression levels can similarly contribute to therapeutic efficacy. This was observed using two independent techniques, however, more pronounced improvement of the phenotype was observed when the inventors dissected and analysed the AAV transduced area of the retina that indeed showed normalization of the number of lipofuscin granules. In conclusion, the invention provides multiple vectors with improved features suitable for clinical application, in particular for the therapy of retinal diseases. In addition, the invention improves the safety and efficacy of multiple vectors which further expand cargo capacity ^{20, 42}.

### REFERENCES

1. Trapani, I, et al (2014). Progress in retinal and eye research 43: 108-128.
2. Boye, SE, Boye, SL, Lewin, AS, and Hauswirth, WW (2013). Molecular therapy : the journal of the American Society of Gene Therapy 21: 509-519.
3. Bainbridge, JW, et al. (2008). The New England journal of medicine 358: 2231-2239.
4. Maguire, AM, et al. (2009). Lancet 374: 1597-1605.
5. Maguire, AM, et al. (2008). The New England journal of medicine 358: 2240-2248.
6. Cideciyan, AV, et al. (2009). Human gene therapy 20: 999-1004.
7. Simonelli, F, et al. (2010). Molecular therapy : the journal of the American Society of Gene Therapy 18: 643-650.
8. Allikmets, R, et al. (1997). Nature genetics 15: 236-246.
9. Molday, RS, and Zhang, K (2010). Progress in lipid research 49: 476-492.
10. Millan, JM, et al. (2011). Journal of ophthalmology 2011: 417217.
11. Hasson, T, et al. (1995). PNAS 92: 9815-9819.
12. Liu, X, Ondek, B, and Williams, DS (1998). Nature genetics 19: 117-118.
13. Gibbs, D, et al. (2010). Investigative ophthalmology & visual science 51: 1130-1135.
14. Trapani, I, Colella, P, Sommella, A, Iodice, C, Cesi, G, de Simone, S, et al. (2014). Effective delivery of large genes to the retina by dual AAV vectors. EMBO molecular medicine 6: 194-211.
15. Duan, D, Yue, Y, and Engelhardt, JF (2001). Molecular therapy : the journal of the American Society of Gene Therapy 4: 383-391.
16. Ghosh, A, Yue, Y, Lai, Y, and Duan, D (2008). Molecular therapy : the journal of the American Society of Gene Therapy 16: 124-130.
17. Dyka, FM, et al., (2014). Human gene therapy methods 25: 166-177.
18. Lopes, VS, et al. (2013). Gene Ther*.*
19. Colella, P, et al. (2014). Gene Ther 21: 450-456.
20. Lostal, W, Kodippili, K, Yue, Y, and Duan, D (2014). Human gene therapy 25: 552-562.
21. Flotte, TR, et al. (1993). The Journal of biological chemistry 268: 3781-3790.
22. Ghosh, A, Yue, Y, and Duan, D (2011). Human gene therapy 22: 77-83.
23. Chiorini, JA, et al., (1999). Journal of virology 73: 1309-1319.
24. Yan, Z, Zak, R, Zhang, Y, and Engelhardt, JF (2005). Journal of virology 79: 364-379.
25. Yan, Z, et al. (2007). Human gene therapy 18: 81-87.
26. Karali, et al. (2010). BMC genomics 11: 715.
27. Kutty, RK, et al. (2010). Molecular vision 16: 1475-1486.
28. Ragusa, M, et al. (2013). Molecular vision 19: 430-440.
29. Sundermeier, TR, and Palczewski, K (2012). Cellular and molecular life sciences : CMLS 69: 2739-2750.
30. Karali, M, et al. (2011). PloS one 6: e22166.
31. Gilon, T, Chomsky, O, and Kulka, RG (1998). The EMBO journal 17: 2759-2766.
32. Bence, NF, Sampat, RM, and Kopito, RR (2001). Science 292: 1552-1555.
33. Bachmair, A, Finley, D, and Varshavsky, A (1986). Science 234: 179-186.
34. Johnson, ES, et al., (1992). The EMBO journal 11: 497-505.
35. Sadis, S, et al., (1995). Molecular and cellular biology 15: 4086-4094.
36. Chiorini, JA, Afione, S, and Kotin, RM (1999). Journal of virology 73: 4293-4298.
37. Tian, W, et al. (2012). PloS one 7: e29551.
38. Wang, Z (2011). Methods in molecular biology 676: 211-223.
39. Mussolino, C, et al. (2011). Gene Ther 18: 637-645.
40. Hendrickson, A, and Hicks, D (2002). Experimental eye research 74: 435-444.
41. Reich, SJ, et al. (2003). Human gene therapy 14: 37-44.
42. Koo, T, et al., (2014). Human gene therapy 25: 98-108.
43. Walters, RW, Bradrick, SS, and Gromeier, M (2010). Rna 16: 239-250.
44. Ricci, EP, et al. (2011). Nucleic acids research 39: 5215-5231.
45. Auricchio, et al. (2001). Human molecular genetics 10: 3075-3081.
46. Gao, G, et al. (2000). Human gene therapy 11: 2079-2091.
47. Young, JE, et al., (2003). Investigative ophthalmology & visual science 44: 4076-4085.
48. Doria, M, et al., (2013). Human gene therapy methods 24: 392-398.
49. Zhang, Y, et al., (2000). Journal of virology 74: 8003-8010.
50. Drittanti, L, et al., (2000). Gene Ther 7: 924-929.
51. Gargiulo, S, et al. (2012). ILAR journal / National Research Council, Institute of Laboratory Animal Resources 53: E70-81.
52. Liang, FQ, et al., (2001). Methods in molecular medicine 47: 125-139.
53. Beltran, et al. (2012) Proc. Natl. Acad. Sci. U. S. A., 109, 2132-2137.
54. Boye, S.E., et al. (2012) Hum. Gene Ther., 23, 1101-1115.
55. Khani, S.C., et al., (2007) Invest. Ophthalmol. Vis. Sci., 48, 3954-3961.
56. Chandler, M.J., et al., (1999) Vet. Ophthalmol., 2, 179-184.
57. Li, A., Zhu, X. and Craft, C.M. (2002) Invest. Ophthalmol. Vis. Sci., 43, 1375-1383.
58. Allocca, M., et al. (2008) J. Clin. Invest., 118, 1955-1964.
59. Parish, C.A., et al., (1998) Proc. Natl. Acad. Sci. U. S. A., 95, 14609-14613.
60. Ben-Shabat, S., et al., (2002) J. Biol. Chem., 277, 7183-7190.
61. Gargiulo, S., et al., (2012) ILAR J, 53, E70-81.
62. Liang, F.Q., et al., (2001) Methods Mol. Med., 47, 125-139.
63. Gargiulo, A., et al. (2009) Mol. Ther., 17, 1347-1354.
64. Manfredi, A., et al. (2013) Hum. Gene Ther., 24, 982-992.
65. Venables VN and Ripley BD. (2002) Modern Applied Statistics with S. Springer Science+Business Media, New York, USA.
66. Li, A., Zhu, X., Brown, B. and Craft, C.M. (2003) Adv. Exp. Med. Biol., 533, 361-368.
67. Li, A., et al. (2003) Invest. Ophthalmol. Vis. Sci., 44, 996-1007.
68. Allocca, M., et al. (2011) Invest. Ophthalmol. Vis. Sci., 52, 5713-5719.
69. Testa, F., et al. (2011) Invest. Ophthalmol. Vis. Sci., 52, 5618-5624.
70. Molday, L.L., Rabin, A.R. and Molday, R.S. (2000) Nat. Genet., 25, 257-258.
71. Sparrow, J.R., Wu, Y., Nagasaki, T., Yoon, K.D., Yamamoto, K. and Zhou, J. (2010) Photochem Photobiol Sci, 9, 1480-1489.
72. Sparrow, J.R. and Duncker, T. (2014) J Clin Med, 3, 1302-1321.
73. Finnemann, S.C., Leung, L.W. and Rodriguez-Boulan, E. (2002) Proc. Natl. Acad. Sci. U. S. A., 99, 3842-3847.
74. Secondi, R., Kong, J., Blonska, A.M., Staurenghi, G. and Sparrow, J.R. (2012) Invest. Ophthalmol. Vis. Sci., 53, 5190-5197.
75. Delori, F.C., Dorey, C.K., Staurenghi, G., Arend, O., Goger, D.G. and Weiter, J.J. (1995) Invest. Ophthalmol. Vis. Sci., 36, 718-729.

The present invention also provides further aspects as defined in the following numbered clauses.
1-A vector system to express the coding sequence of a gene of interest in a cell, said coding sequence comprising a first portion and a second portion, said vector system comprising:
   c) a first vector comprising:
      - said first portion of said coding sequence (CDS1),
      - a first reconstitution sequence; and
   d) a second vector comprising:
      - said second portion of said coding sequence (CDS2),
      - a second reconstitution sequence,
         wherein said first and second reconstitution sequences are selected from the group of:
         i] the first reconstitution sequence consists of the 3' end of said first portion of the coding sequence and the second reconstitution sequence consists of the 5'end of said second portion of the coding sequence, said first and second reconstitution sequences being overlapping sequences; or
         ii] the first reconstitution sequence comprises a splicing donor signal (SD) and the second reconstitution sequence comprises a splicing acceptor signal (SA), optionally each one of first and second reconstitution sequence further comprises a recombinogenic sequence,
   characterized by the fact that either one or both of the first and second vector further comprises a nucleotide sequence of a degradation signal said sequence being located in case of i) at the 3' end of the CDS1 and/or at the 5' end of the CDS2 and in case of ii) in 3' position relative to the SD and/or in 5' position relative to the SA.
2- The vector system according to clause 1, wherein both of the first and second vector further comprise said nucleotide sequence of a degradation signal, wherein the nucleotide sequence of the degradation signal in the first vector is identical to or differs from that in the second vector.
3- The vector system according to any of clauses 1 or 2, wherein the first reconstitution sequence comprises a splicing donor signal (SD) and a recombinogenic region in 3' position relative to said SD, the second reconstitution sequence comprises a splicing acceptor signal (SA) and a recombinogenic sequence in 5' position relative to the SA; wherein said nucleotide sequence of a degradation signal is localized at the 5' end and/or at the 3' end of the nucleotide sequence of the recombinogenic region of either one or both of the first and second vector.
4- The vector system according to any one of previous clauses, wherein the nucleotide sequence of the degradation signal is selected from: one or more protein ubiquitination signals, one or more microRNA target sequences, and/or one or more artificial stop codons.
5- The vector system according to any one of previous clauses, wherein the nucleotide sequence of the degradation signal comprises or consists of a sequence encoding a sequence selected from CL1 SEQ ID No. 1, CL2 SEQ ID No. 2, CL6 SEQ ID No. 3, CL9 SEQ ID No. 4, CL10 SEQ ID No. 5, CL11 SEQ ID No. 6, CL12 SEQ ID No. 7, CL15 SEQ ID No. 8, CL16 SEQ ID No. 9, SL17 SEQ ID No. 10, or PB29 (SEQ ID No. 14 or SEQ ID No. 15); or wherein the nucleotide sequence of the degradation signal comprises or consists of a sequence selected from miR-204 SEQ ID No. 11, miR-124 SEQ ID No. 12 or miR-26a SEQ ID No. 13.
6- The vector system according to any one of previous clauses, wherein the nucleotide sequence of the degradation signal of the first vector comprises or consists of a sequence encoding CL1 SEQ ID No. 1 or comprises or consists of SEQ ID No. 16 or comprises or consists of miR-204 SEQ ID No. 11 and miR-124 SEQ ID No. 12, preferably comprises three copies of miR 204 SEQ ID No. 11 and three copies of miR 124 SEQ ID No. 12, or comprises or consists of miR-26a SEQ ID No. 13, preferably comprises four copies of miR-26a SEQ ID No. 13.
7- The vector system according to any one of previous clauses, wherein the nucleotide sequence of the degradation signal of the second vector comprises or consists of a sequence encoding PB29 (SEQ ID No. 14 or SEQ ID No. 15) or comprises or consists of SEQ ID No. 19 or SEQ ID No. 20, preferably the degradation signal of the second vector comprises or consists of a sequence encoding three copies of PB29 of SEQ ID No. 14 or SEQ ID No. 15.
8- The vector system according to any one of previous clauses, wherein the first vector further comprises a promoter sequence operably linked to the 5'end portion of said first portion of the coding sequence (CDS1).
9- The vector system according to any one of previous clauses, wherein both of the first vector and the second vector further comprise a 5'-terminal repeat (5'-TR) nucleotide sequence and a 3'-terminal repeat (3'-TR) nucleotide sequence, preferably the 5'-TR is a 5'-inverted terminal repeat (5'-ITR) nucleotide sequence and the 3'-TR is a 3'-inverted terminal repeat (3'-ITR) nucleotide sequence, preferably the ITRs derive from the same virus serotype or from different virus serotypes, preferably the virus is an AAV.
10- The vector system according to any one of previous clause, wherein the recombinogenic sequence is selected from the group consisting of: AK GGGATTTTGCCGATTTCGGCCTATTGGTTAAAAAATGAGCTGATTTAACAAAAATTT AACGCGAATTTTAACAAAAT(SEQ ID No. 22) or GGGATTTTTCCGATTTCGGCCTATTGGTTAAAAAATGAGCTGATTTAACAAAAATTT AACGCGAATTTTAACAAAAT (SEQ ID NO. 23), AP1 (SEQ ID NO. 24), AP2 (SEQ ID NO. 25), and AP (SEQ ID NO. 26).
11- The vector system according to any one of previous clause, wherein the coding sequence is split into the first portion and the second portion at a natural exon-exon junction.
12- The vector system according to any one of previous clause, wherein the splicing donor signal comprises or consists essentially of a sequence that is at least 70%, 75%, 80%, 85%, 90%, 95% or 100 % identical to GTAAGTATCAAGGTTACAAGACAGGTTTAAGGAGACCAATAGAAACTGGGCTTGTC GAGACAGAGAAGACTCTTGCGTTTCT (SEQ ID No. 27).
13- The vector system according to any one of previous clause, wherein the splicing acceptor signal comprises or consists essentially of a sequence that is at least 70%, 75%, 80%, 85%, 90%, 95% or 100 % identical to GATAGGCACCTATTGGTCTTACTGACATCCACTTTGCCTTTCTCTCCACAG (SEQ ID No. 28)
14- The vector system according to any one of previous clause, wherein the first vector further comprises at least one enhancer nucleotide sequence, operably linked to the coding sequence.
15- The vector system according to any one of previous clause wherein the coding sequence encodes a protein able to correct a retinal degeneration.
16- The vector system according to any one of clause 1 to 14 wherein the coding sequence encodes a protein able to correct Duchenne muscular dystrophy, cystic fibrosis, hemophilia A and dysferlinopathies.
17- The vector system according to any one of clause 1 to 15, wherein the coding sequence is the coding sequence of a gene selected from the group consisting of: ABCA4, MYO7A, CEP290, CDH23, EYS, PCDH15, CACNA1, SNRNP200, RP1, PRPF8, RP1L1, ALMS1, USH2A, GPR98, HMCN1.
18- The vector system according to any one of clause 1 to 14 and 16, wherein the coding sequence is the coding sequence of a gene selected from the group consisting of: DMD, CFTR, F8 and DYSF.
19- The vector system according to any one of previous clauses, wherein the first vector does not comprise a poly-adenylation signal nucleotide sequence.
20- The vector system according to any one of previous clauses comprising:
   c) a first vector comprising in a 5'-3' direction:
      - a 5'-inverted terminal repeat (5'-ITR) sequence;
      - a promoter sequence;
      - a 5' end portion of a coding sequence of a gene of interest (CDS1), said 5'end portion being operably linked to and under control of said promoter;
      - a nucleotide sequence of a splicing donor signal;
      - a nucleotide sequence of a recombinogenic region; and
      - a 3'-inverted terminal repeat (3'-ITR) sequence; and
   d) a second vector comprising in a 5'-3' direction:
      - a 5'-inverted terminal repeat (5'-ITR) sequence;
      - a nucleotide sequence of a recombinogenic region;
      - a nucleotide sequence of a splicing acceptor signal;
      - the 3'end of the coding sequence (CDS2);
      - a poly-adenylation signal nucleotide sequence; and
      - a 3'-inverted terminal repeat (3'-ITR) sequence,
   characterized by further comprising a nucleotide sequence of a degradation signal, said sequence being localized at 5' end or 3' end of the nucleotide sequence of the recombinogenic region of either one or both of the first and second vector.
21- The vector system according to any one of previous clauses wherein said first and second vector is independently a viral vector, preferably an adeno viral vector or adeno-associated viral (AAV) vector, preferably said first and second adeno-associated viral (AAV) vectors are selected from the same or different AAV serotypes, preferably the adeno-associated virus is selected from the serotype 2, the serotype 8, the serotype 5, the serotype 7 or the serotype 9.
22- The vector system according to any one of previous clauses further comprising a third vector comprising a third portion of said coding sequence (CDS3) and a reconstitution sequence, wherein the second vector comprises two reconstitution sequences, each reconstitution sequence located at each end of CDS2.
23- The vector system of clause 22 wherein the third vector further comprises at least one nucleotide sequence of a degradation signal.
24- The vector system according to any one of clauses 1-21, wherein the second vector further comprises a poly-adenylation signal nucleotide sequence linked to the 3'end portion of said coding sequence (CDS2).
25- A host cell transformed with the vector system according to any one of previous clause.
26- The vector system according to any one of clause 1 to 24 or the host cell according to clause 25 for medical use.
27- The vector system according to any one of clause 1 to 24 or the host cell according to clause 25 for use in gene therapy.
28- The vector system according to clause 1-15 and 17 or the host cell according to clause 25 for use in the treatment and/or prevention of a pathology or disease characterized by a retinal degeneration.
29- The vector system or the host cell for use according to clause 28 wherein the retinal degeneration is inherited.
30- The vector system or the host cell for use according to clause 28 or 29 wherein the pathology or disease is selected from the group consisting of: retinitis pigmentosa (RP), Leber congenital amaurosis (LCA), Stargardt disease (STGD), Usher disease (USH), Alstrom syndrome, congenital stationary night blindness (CSNB), macular dystrophy, occult macular dystrophy, a disease caused by a mutation in the *ABCA4* gene.
31- The vector system according to any one of clause 1-14, 16 and 18 or the host cell according to clause 25 for use in the prevention and/or treatment of Duchenne muscular dystrophy, cystic fibrosis, hemophilia A and dysferlinopathies.
32- A pharmaceutical composition comprising the vector system according to any one of clause 1 to 24 or the host cell according to clause 25 and pharmaceutically acceptable vehicle.
33- A method for treating and/or preventing a pathology or disease characterized by a retinal degeneration comprising administering to a subject in need thereof an effective amount of the vector system according to any one of clause 1 to 15 and 17, the host cell according to clause 25 or the pharmaceutical composition according to clause 32.
34- A method for treating and/or preventing Duchenne muscular dystrophy, cystic fibrosis, hemophilia A or dysferlinopathies comprising administering to a subject in need thereof an effective amount of the vector system according to any one of clause 1 to 14, 16 and 18, the host cell according to clause 25 or the pharmaceutical composition according to clause 32.
35- Use of a nucleotide sequence of a degradation signal in a vector system to decrease expression of a protein in truncated form.
36- A method for decreasing expression of a protein in truncated form comprising inserting a nucleotide sequence of a degradation signal in one or more vector of a vector system.

## Claims

1. A vector system to express the coding sequence of a gene of interest in a cell, said coding sequence comprising a first portion and a second portion, said vector system comprising:
(a) a first vector comprising said first portion of said coding sequence (CDS1), and a first reconstitution sequence; and
(b) a second vector comprising said second portion of said coding sequence (CDS2), and a second reconstitution sequence,
wherein said first and second reconstitution sequences are selected from the group consisting of:
(i) the first reconstitution sequence consists of the 3' end of said first portion of the coding sequence and the second reconstitution sequence consists of the 5' end of said second portion of the coding sequence, said first and second reconstitution sequences being overlapping sequences; or
(ii) the first reconstitution sequence comprises a splicing donor signal (SD) and the second reconstitution sequence comprises a splicing acceptor signal (SA), and optionally each one of the first and second reconstitution sequence further comprises a recombinogenic sequence,
**characterized by** the fact that either one or both of the first and second vector further comprises a nucleotide sequence of a degradation signal said sequence being located in the case of (i) at the 3' end of the CDS1 and/or at the 5' end of the CDS2 and in the case of (ii) in 3' position relative to the SD and/or in 5' position relative to the SA.

2. The vector system of claim 1, wherein (a) both of the first and second vector further comprise said nucleotide sequence of a degradation signal, wherein the nucleotide sequence of the degradation signal in the first vector is identical to or differs from that in the second vector; and/or (b) the first reconstitution sequence comprises a splicing donor signal (SD) and a recombinogenic region in 3' position relative to said SD, the second reconstitution sequence comprises a splicing acceptor signal (SA) and a recombinogenic sequence in 5' position relative to the SA, wherein said nucleotide sequence of a degradation signal is localized at the 5' end and/or at the 3' end of the nucleotide sequence of the recombinogenic region of either one or both of the first and second vector.

3. The vector system of claim 1 or 2, wherein the nucleotide sequence of the degradation signal is selected from: one or more protein ubiquitination signals, one or more microRNA target sequences, and/or one or more artificial stop codons.

4. The vector system of any preceding claim, wherein:
(a) the nucleotide sequence of the degradation signal comprises or consists of a sequence encoding a sequence selected from SEQ ID NO: 1 (CL1), SEQ ID NO: 2 (CL2), SEQ ID NO: 3 (CL6), SEQ ID NO: 4 (CL9), SEQ ID NO: 5 (CL10), SEQ ID NO: 6 (CL11), SEQ ID NO: 7 (CL12), SEQ ID NO: 8 (CL15), SEQ ID NO: 9 (CL16), SEQ ID NO: 10 (SL17), SEQ ID NO: 14 or SEQ ID NO: 15 (PB29); and/or the nucleotide sequence of the degradation signal comprises or consists of SEQ ID NO: 16; and/or
(b) the nucleotide sequence of the degradation signal comprises or consists of a sequence selected from SEQ ID NO: 11, SEQ ID NO: 12 or SEQ ID NO: 13.

5. The vector system of any preceding claim, wherein the first vector further comprises a promoter sequence operably linked to the 5' end portion of said first portion of the coding sequence (CDS1).

6. The vector system of any preceding claim, wherein the recombinogenic sequence is selected from the group consisting of: GGGATTTTGCCGATTTCGGCCTATTGGTTAAAAAATGAGCTGATTTAACAAAAATTTAAC GCGAATTTTAACAAAAT (AK; SEQ ID NO: 22), GGGATTTTTCCGATTTCGGCCTATTGGTTAAAAAATGAGCTGATTTAACAAAAATTTAAC GCGAATTTTAACAAAAT (AK; SEQ ID NO: 23), SEQ ID NO: 24 (AP1), SEQ ID NO: 25 (AP2), and SEQ ID NO: 26 (AP).

7. The vector system of any preceding claim, wherein the splicing donor signal comprises or consists essentially of a sequence that is at least 70%, 75%, 80%, 85%, 90%, 95% or 100 % identical to GTAAGTATCAAGGTTACAAGACAGGTTTAAGGAGACCAATAGAAACTGGGCTTGTCGAG ACAGAGAAGACTCTTGCGTTTCT (SEQ ID NO: 27); and/or wherein the splicing acceptor signal comprises or consists essentially of a sequence that is at least 70%, 75%, 80%, 85%, 90%, 95% or 100 % identical to GATAGGCACCTATTGGTCTTACTGACATCCACTTTGCCTTTCTCTCCACAG (SEQ ID No: 28).

8. The vector system of any preceding claim, wherein the coding sequence:
(a) encodes a protein able to correct a retinal degeneration;
(b) encodes a protein able to correct Duchenne muscular dystrophy, cystic fibrosis, hemophilia A and dysferlinopathies;
(c) is the coding sequence of a gene selected from the group consisting of: ABCA4, MYO7A, CEP290, CDH23, EYS, PCDH15, CACNA1, SNRNP200, RP1, PRPF8, RP1L1, ALMS1, USH2A, GPR98, HMCN1; and/or
(d) is the coding sequence of a gene selected from the group consisting of: DMD, CFTR, F8 and DYSF.

9. The vector system of any preceding claim, wherein the full length coding sequence is obtained when the vectors of the vector system are introduced into a cell.

10. The vector system of any preceding claim, wherein the vector system comprises:
(a) a first vector comprising in a 5'-3' direction: a 5'-inverted terminal repeat (5'-ITR) sequence; a promoter sequence; a 5' end portion of a coding sequence of a gene of interest (CDS1), said 5' end portion being operably linked to and under control of said promoter; a nucleotide sequence of a splicing donor signal; a nucleotide sequence of a recombinogenic region; and a 3'-inverted terminal repeat (3'-ITR) sequence; and
(b) a second vector comprising in a 5'-3' direction: a 5'-inverted terminal repeat (5'-ITR) sequence; a nucleotide sequence of a recombinogenic region; a nucleotide sequence of a splicing acceptor signal; the 3' end of the coding sequence (CDS2); a poly-adenylation signal nucleotide sequence; and a 3'-inverted terminal repeat (3'-ITR) sequence,
**characterized by** further comprising a nucleotide sequence of a degradation signal, said sequence being localized at 5' end or 3' end of the nucleotide sequence of the recombinogenic region of either one or both of the first and second vector.

11. The vector system of any preceding claim, wherein the vector system further comprises a third vector comprising a third portion of said coding sequence (CDS3) and a reconstitution sequence, wherein the second vector comprises two reconstitution sequences, each reconstitution sequence located at each end of CDS2, optionally wherein the third vector further comprises at least one nucleotide sequence of a degradation signal.

12. A vector, wherein the vector is the first vector, second vector or third vector as defined in any preceding claim.

13. A host cell comprising the vector system or vector according to any preceding claim.

14. The vector system, vector or host cell of any preceding claim for use in therapy, optionally for use in the treatment and/or prevention of:
(a) a genetic disease;
(b) a pathology or disease **characterized by** a retinal degeneration, optionally wherein the retinal degeneration is inherited, optionally wherein the pathology or disease is selected from the group consisting of: retinitis pigmentosa (RP), Leber congenital amaurosis (LCA), Stargardt disease (STGD), Usher disease (USH), Alstrom syndrome, congenital stationary night blindness (CSNB), macular dystrophy, occult macular dystrophy, and a disease caused by a mutation in the *ABCA4* gene; or
(c) Duchenne muscular dystrophy, cystic fibrosis, hemophilia A and dysferlinopathies.

15. Use of a nucleotide sequence of a degradation signal in a vector system for decreasing expression of a protein in truncated form.
